# EUROPEAN PATENT SPECIFICATION

(11) **EP 2 858 649 B1**
(45) Date of publication and mention of the grant of the patent: **23.10.2019**
(21) Application number: 13805005.9
(22) Date of filing: 06.06.2013
(51) Int. Cl.: A61K 31/496, A61K 31/198, A61P 25/16

(54) **TREATMENT OF MOTOR AND MOVEMENT DISORDER SIDE EFFECTS ASSOCIATED WITH PARKINSON'S DISEASE TREATMENTS**
BEHANDLUNG VON NEBENWIRKUNGEN MOTORISCHER UND BEWEGUNGSSTÖRUNG IM ZUSAMMENHANG MIT MORBUS-PARKINSON-BEHANDLUNGEN
TRAITEMENT DES EFFETS SECONDAIRES DES TROUBLES MOTEURS ET DE LA DYSKINÉSIE ASSOCIÉS AUX TRAITEMENTS ANTIPARKINSONIENS

(30) Priority: 11.06.2012 US 201261658401 P
(43) Date of publication of application: 15.04.2015
(73) Proprietor: Psychogenics Inc., Tarrytown, New York 10591 (US)
(72) Inventor: LEAHY, Emer, Pound Ridge, New York 10576 (US); SHANKAR, Bavani, Nanuet, New York 10954 (US)
(74) Representative: Gurney, Steven
(86) International application number: PCT/US2013/044509
(87) International publication number: WO 2013/188210

(56) References cited:
- WO-A1-2009/156330
- WO-A1-2010/063486
- WO-A1-2012/048710
- US-A1- 2011 183 995

## Description

### FIELD OF THE INVENTION

This invention relates generally to the administration of eltoprazine or a pharmaceutically acceptable acid addition salt thereof to human adult patients in need thereof in order to prevent, attenuate and/or treat movement disorders or motor disorder side effects associated with administration of levodopa (L-DOPA) or a dopamine-related drug to a human adult patient having Parkinson's disease. In particular, the invention relates to preventing and treating L-DOPA-induced dyskinesia (LID) by administering eltoprazine, either alone or in combination with other compounds.

### BACKGROUND OF THE INVENTION

Parkinson's disease is a chronic, progressive, hypokinetic neurodegenerative disorder characterized by impaired voluntary movement (See, Dale and Federman (eds.), WebMD Scientific American Medicine, NY: WebMD Corporation, Chapter 11, Section 15, pp. 1-21,2001; Lang and Lozano, N Engl J Med, 339: 1044, 1998; and Lang and Lozano, N Engl J Med, 339: 1130, 1998). Parkinson's disease occurs at least as a result of the death of dopamine-producing neurons in the substantia nigra of the midbrain. Dopamine is a neurotransmitter, or chemical messenger, that transports signals to the parts of the brain that control movement initiation and coordination. The loss of dopamine in the brain is associated with multiple primary symptoms including, for example, tremor of the hands, arms, legs, jaw, and face; rigidity or stiffness of the limbs and trunk; bradykinesia or slowness of movement; dyskinesia; and postural instability or impaired balance and coordination. The disease is also associated with dementia, sleep disturbances and cognitive confusion.

Parkinson's disease afflicts more than one million persons in the United States alone (Lang and Lozano, supra, 1998), with approximately 50,000 new cases diagnosed each year. It is generally a disease of late middle age, with typical onset occurring at about age 60. About five percent of patients, however, have early-onset disease and are younger than 40 when symptoms begin.

Most reported treatment strategies for PD focus on symptom control through one or more of medication, surgery, and physical therapy. Administration of the dopamine precursor, L-DOPA (L-3,4-dihydroxyphenylalanine; levodopa) is reported as the most effective and most commonly used treatment for PD, as it reverses the motor deficits associated with PD more so than dopamine agonists (Goodman & Gillman's The Pharmacolgoical Basis of Therapeutics, 11th edition, L. Brunton editor, The McGraw Hill Company, 2006). Unfortunately, L-DOPA can cause debilitating side effects (LeWitt and Nyholm Neurology, 62:S9-S16, 2004), including severe nausea, vomiting, and psychosis. Moreover, with prolonged use, patients frequently experience other side effects such as dyskinesia, or abnormal, excessive movements in many patients, which can interfere with the management of PD (see, *e.g.,* Baldessarini RJ., Am. J. Psychiatry, 137: 1163-72 (1980); Samii et al., Lancet, 363(9423): 1783-93(2004)).

Serotonin neurons are reported to play a role in the development of L-DOPA-induced dyskinesia (LID), and specifically, 5-HT₁ₐ and 5-HT_{1b} receptors are thought to be involved (see, *e.g.,* Carta et al., Brain, 130(7): 1819-33 (2007); and U.S. published application 2007/0249621). In rats, activation of 5-HT₁ₐ and 5-HT_{1b} receptors, either separately, or in combination, was reported to reduce LID (Carta *et al.,* 2007). When administered together at certain low doses, 5-HT₁ₐ and 5-HT_{1b} receptor agonists were stated to block LID in rats (Carta *et al.,* 2007). WO2010/063486 to Merz et al.*,* WO 2009/156380 to Bjorklund et al.; U.S. patent publication 2007/0249621 to Wolf et. al.; U.S. patent publication 2010/0179171 to Wolf et. al.; and European Patent Application No. 2193794 to Valastro et al. relate to the use of eltoprazine to treat LID in rats.

The binding profile of eltoprazine as reported by Sijbesma (Sijbesma, H. et al., European Journal of Pharmacology, 187(2): 209-223, (1990)) shows the compound to be a selective 5-HT₁ ligand (selective with respect to all receptors other than 5-HT₁) and similar to serotonin in many respects except for a lower affinity for the 5-HT_{1d} receptor. The literature reports that eltoprazine acts as a mixed 5-HT₁ₐ/5-HT_{1b} receptor agonist with roughly equipotent affinity for each of these receptors (Schipper et al., Drug Metabol Drug Interact, 8(1-2):85-114, (1990)). Eltoprazine has no relevant affinity for dopamine receptors. Among the 5-HT receptors, the 5-HT_{1b} receptor is located as an autoreceptor on axon terminals and is responsible for inhibiting neurotransmitter release, whereas it is also located postsynaptically as a heteroreceptor on axons and terminals of non-serotonergic neurons inhibiting their activity (Clark and Neumaier, Psychopharmacol Bul, 35(4):170-85, (2001)).

There exists a need to develop noninvasive treatments which can effectively prevent, attenuate, control, and treat the motor disorder side effects or movement disorders associated with drug treatments for PD. There is a need to prevent, attenuate, and treat L-DOPA-induced dyskinesia (LID), as well as to prevent, attenuate, and treat Parkinson's disease (PD). In particular, there is a need to develop strategies that target serotonin receptors, including 5-HT₁ₐ and 5-HT_{1b} receptors, either in combination or separately, for the prevention, attenuation and treatment of movement disorders, such as L-DOPA-induced dyskinesia, associated with Parkinson's disease treatments. There is also a need to develop treatment strategies for controlling the symptoms of Parkinson's disease and other movement disorders and effective pharmaceutical compounds and dosages for treating these symptoms. In addition, there is a need for specific therapy regimens that maximize the efficacy, and reduce the side effects of existing medicines, for Parkinson's disease.

### SUMMARY OF INVENTION

This invention contemplates eltoprazine or a pharmaceutically acceptable acid addition salt thereof for use in a method of preventing, attenuating and/or treating a movement disorder or a motor disorder side effect associated with administration of levodopa or a dopamine-related drug to a human adult patient having Parkinson's disease; the method comprising administering to said human adult patient having Parkinson's disease in need of treatment eltoprazine or a pharmaceutically acceptable acid addition salt thereof at a dose range of 5 mg/day to 7.5 mg/day. The inventors have determined that certain doses of eltoprazine are efficacious in reducing LID in human adult patients, specifically when eltoprazine is administered to Parkinson's patients, at doses of 5 mg/day to 7.5 mg/day, particularly 5 mg/day, 7 mg/day and 7.5 mg/day. Eltoprazine administration did not affect the efficacy of levodopa treatment, and was not associated with any serious adverse events.

In a further embodiment, there is provided eltoprazine or a pharmaceutically acceptable acid addition salt thereof for use in a method of preventing, attenuating and/or treating levodopa-induced dyskinesia (LID), the method comprising administering to a human adult patient having Parkinson's disease in need of treatment eltoprazine or a pharmaceutically acceptable acid addition salt thereof at a dose range of 5 mg/day to 7.5 mg/day.

The dopamine-related drugs encompassed by the invention include drugs that increase the activity of the dopamine receptor, including dopamine agonists and partial agonists, whether acting directly or indirectly, as well as dopamine precursors, such as L-DOPA. In preferred embodiments, the invention encompasses preventing, attenuating, and/or treating motor disorder side effects, including but not limited to dyskinesia, that are associated with L-DOPA therapy in Parkinson's patients (L-DOPA-induced dyskinesia, LID). The methods of the invention comprise administering to a human adult patient in need thereof a therapeutic dose of a compound having agonist activity at both the 5-HT₁ₐ and 5-HT_{1b} receptors (eltoprazine)

The invention encompasses many possible administration and dosage regimes, with administration strategies including, but not limited to, administration of eltoprazine before or after initiation of L-DOPA or other dopamine-related drug administration, and administration of eltoprazine before or after development of motor disorder side effects. Dosage strategies include therapeutic and sub-therapeutic dosages of L-DOPA or other dopamine-related drug. In certain embodiments, this invention encompasses a reduction in the dosage of L-DOPA or other dopamine-related drug after administration of eltoprazine. Particularly preferred dosages of eltoprazine, in humans, are 5 mg/day and 7.5 mg/day. Administration schedules may also be altered to achieve a therapeutically effective concentration of compound to treat the disorder or symptoms described herein. In some embodiments, for example, the compound may be administered once per day, twice per day, thrice per day, 4 times per day, 5 times per day, 7 times per day or 10 times per day.

Kits comprising one or more of the following are also encompassed by the invention described herein: eltoprazine or a pharmaceutically acceptable acid addition salt thereof, additional compounds, L-DOPA or other or other dopamine-related drug, and instructions for administration. Non-limiting examples of such kits include a kit comprising eltoprazine, and a kit comprising eltoprazine or a pharmaceutically acceptable acid addition salt thereof, L-DOPA, a DDCI inhibitor and/or COMT inhibitor, and optionally any other compound described herein, plus instructions for administration.

### BRIEF DESCRIPTION OF THE FIGURES

**FIG. 1****:** shows the efficacy of eltoprazine in treating levodopa-induced dyskinesia patients (per protocol population), measured using Clinical Dyskinesia Rating Scales (CDRS).
**FIG. 2****:** shows that there is no significant change in the Unified Parkinson's Disease Rating Scales-III (UPDRS-III) of parkinsonian symptoms after eltoprazine administration, indicating that eltoprazine does not adversely interfere with the levodopa efficacy in Parkinson's patients.

### DETAILED DESCRIPTION

This invention encompasses preventing, attenuating, and/or treating motor disorder side effects in a human adult patient with PD, including but not limited to dyskinesia or other motor disorder side effects or movement disorders, that are associated with dopamine-related drugs, in addition to preventing, attenuating, and/or treating PD. The method of the invention comprises administering to a human adult patient in need thereof a therapeutic dose of eltoprazine or a pharmaceutically acceptable acid addition salt thereof. Optionally in combination with targeting the 5-HT₁ₐ and 5-HT_{1b} receptors, the invention encompasses administering to the human adult patient in need thereof a compound that targets the same, a similar, or a different drug pathway, one that is useful in treating L-DOPA-induced dyskinesia (LID) or other movement disorders or motor disorder side effects associated with dopamine-related drugs, one that is useful in treating PD, or one that is useful for treating both LID or other movement disorders, or motor disorder side effects associated with dopamine-related drugs and PD. This additional compound may also be useful such that the effective dose of L-DOPA or other dopamine-related drug that is necessary to treat PD is reduced. In some embodiments, the invention contemplates administering to the human adult patient in need thereof L-DOPA, in addition to a compound that targets the same, a similar, or a different drug pathway, one that is useful in treating L-DOPA-induced dyskinesia (LID), one that is useful in treating PD, or one that is useful for treating both LID or other movement disorders or motor disorder side effects associated with dopamine-related drugs and PD.

The dopamine-related drugs encompassed by the invention include drugs that increase the activity of the dopamine receptor, including dopamine agonists and partial agonists, whether acting directly or indirectly, as well as dopamine precursors, such as L-DOPA. In a preferred embodiment, the dopamine-related drug treatment is L-DOPA therapy.

In other embodiments, the dopamine-related drug may be a dopamine receptor agonist, including, but not limited to bromocriptine, pergolide, cabergoline, apomorphine, and lisuride, or a non-ergoline dopamine agonist, including, but not limited to ropinirole or pramipexole.

In yet other embodiments, the methods of the invention encompass preventing, attenuating, and/or treating any of the motor disorder side effects described herein, associated with a combination of dopamine-related drug treatments, such as a combination of a dopamine precursors, a combination of dopamine agonists or partial agonists, and a combination of one or more dopamine precursors and one or more dopamine agonists or partial agonists.

Dopamine precursors such as L-DOPA are often administered with a DOPA decarboxylase inhibitor (DDCI) (also known as aromatic L-amino acid decarboxylase inhibitors (AAADI)). Non-limiting examples of such compounds contemplated by the invention include benserazide (Madopar, Prolopa, Modopar, Madopark, Neodopasol, and EC-Doparyl); carbidopa (Lodosyn, Sinemet, Parcopa, and Atamet); and Methyldopa (Aldomet, Aldoril, Dopamet, and Dopegyt).

In addition to DDCIs, L-DOPA or other dopamine precursors are also often administered with compounds that inhibit the action of catechol-O-methyl transferase (COMT inhibitors). Non-limiting examples of COMT inhibitors contemplated by the invention are entacapone, tolcapone, and nitecapone.

The methods of the invention, therefore, encompass preventing, attenuating, and/or treating any of the motor disorder side effects described herein, associated with L-DOPA treatment or treatment with another dopamine-related drug, L-DOPA treatment or other dopamine-related drug treatment in combination with DDCI (AAADI) treatment, L-DOPA treatment or other dopamine-related drug treatment in combination with COMT inhibitors, and L-DOPA treatment or other dopamine-related drug treatment in combination with DDCI (AAADAI) treatment and COMT inhibitors.

In one embodiment, the methods of the invention encompass preventing, attenuating, and/or treating any of the motor disorder side effects described herein, associated with administering the combination of carbidopa, levodopa, and entacapone. In one embodiment, the combination of carbidopa, levodopa, and entacapone is administered as Stalevo.

In another embodiment, the methods of the invention encompass preventing, attenuating, and/or treating PD itself, including the movement disorders associated with PD.

The compound having agonist activity at both the 5-HT₁ₐ and 5-HT_{1b} receptors is eltoprazine or a pharmaceutically acceptable salt thereof.

In one embodiment, this invention provides a method of prevention, attenuation, and/or treatment of motor disorder side effects, including but not limited to dyskinesia, that are associated with L-DOPA therapy or other dopamine-related drugs in Parkinson's patients comprising administering to a human adult patient in need thereof a therapeutic dose of eltoprazine or a pharmaceutically acceptable acid addition salt thereof.

In one preferred embodiment, the invention provides a method of preventing, attenuating, and/or treating Parkinson's disease, comprising administering to a human adult patient in need thereof a dopamine-related drug and most preferably, L-DOPA, in combination with a therapeutic dose of eltoprazine or a pharmaceutically acceptable acid addition salt thereof.

In some embodiments, the invention provides methods for treating one or more symptoms of Parkinson's disease. Examples of such symptoms include but are not limited to dyskinesia, hyperkinesia, speech changes, loss of facial expression, cognitive dysfunction, mood swings, emotionallability, euphoria, bipolar syndrome, anxiety, aphasia, dysphasia, or disturbances, dementia or confusion, depression, fear, anxiety, memory difficulties, slowed thinking, sexual dysfunction, fatigue, aching, and loss of energy.

For all the conditions described herein, one of ordinary skill in the art will appreciate how to determine the presence or absence of characteristic symptoms and also how to diagnose these conditions. A number of criteria for diagnosing disease are useful for characterizing these conditions such as for example, NINCDS-ADRDA criteria (McKhann *et al.,* 1984), the ICD-IO criteria (World Health Organization, 1992), and/or the DSM-IV criteria (American Psychiatric Association, 1994). Other manuals useful in diagnosing the conditions described herein include for example, but are not limited to Oppenheimer's Diagnostic Neuropathology: A Practice Manual (Esiri and Perl, 2006, Hodder Amold, London.); Harrison's Principles of Internal Medicine (Ed. Kasper et al, 16th Ed. 2005 McGraw Hill, Columbus, Ohio); Goetz: Textbook of Clinical Neurology (Eds. Goetz, Pappert, 2nd Ed. 2003, W.B. Saunders, Philadelphia, Pa.). One of ordinary skill will be aware of other such manuals routinely used in the art to diagnose these conditions.

Eltoprazine (1-(2,3-dihydro-1,4-benzodioxanyl-5-yl) piperazine) is used with this invention, including pharmaceutically acceptable salts thereof, and preferably HCl. This invention also includes the use of prodrugs of the compounds of the formulas provided, specifically derivatives of the compounds of the formulas that are inactive but are converted to an active form in the body following administration.

Eltoprazine and processes for its synthesis are known in the art and is described in U.S. Pat. No. 4,833,142; U.S. Pat. No. 5,424,313; European Patent No. 189,612; and European Patent No. 138,280. Eltoprazine is commercially available, for example, through Tocris Bioscience (Ellisville, Missouri).

In one embodiment, eltoprazine or a pharmaceutically acceptable acid addition salt thereof is administered in combination with one or more additional compound(s). The additional compound(s) may have actions that are similar to, synergistic to, or different than eltoprazine and/or its related compound(s). Non-limiting examples of additional compounds include (mTOR) complex 1 (mTORC1) inhibitors (e.g., rapamycin), 5-HT1a agonists (e.g., buspirone, gepirone, tandospirone, flesinoxan, ziprasidone, and EMD128130 (sarizotan)), 5-HT1a/2a/alpha-2 antagonists (e.g., mirtazapine), 5-HT2a inverse agonists (e.g., ACP103 (pimavanserin), nelotanserin, eplivanserin, AC-90179 [2-(4-Methoxyphenyl)-N-(4-methyl-benzyl)-N-(1-methyl-piperidin-4-yl)-acetamide Hydrochloride]), adenosine A₂ₐ receptor antagonists (*e.g*., KW-6002 (istradefylline), SYN-115 (4-Hydroxy-N-[4-methoxy-7-(4-morpholinyl)-2-benzothiazolyl]-4-methyl-1-piperidinecarboxamide, Synosia Therapeutics), SCH420814 (preladenant), ZM-241,385 (4-(2-[7-amino-2-(2-furyl) [1,2,4]-triazolo[2,3-a][1,3,5]triazin-5-yl amino]ethyl) phenol), VER-7835 (2-amino-6-(furan-2-yl)-N-(thiophen-2-ylmethyl)-9H-purine-9- carboxamide), MSX-3 (3-(3-hydroxypropyl)-7-methyl-8-(m-methoxystyryl)-1-propargylxanthine), ST-1535 (2-butyl-9-methyl-8-(2H-1,2,3-triazol 2-yl)-9 H-purin-6-ylamine)), alpha(1) adrenoceptor antagonists (e.g., HEAT (2-[[beta-(-4-hydroxyphenyl)ethyl]aminomethyl]-1-tetralone), alfuzosin, doxazosin, moxisylyte, prazosin, trimazosin), alpha-2 adrenergic receptor antagonists (*e.g.,* idazoxan, JP1730 (fipamezole), atipamezole, mianserin, phentolamine, tolazoline), AMPA antagonists (*e.g.,* E2007 (perampanel)), AMPA/kainate antagonists (*e.g.,* LY300164 [7-acetyl-5-(4-aminophenyl)-8,9-dihydro-8-methyl-7H-1,3-dioxolo(4,5H)-2,3-benzodiazepine]), anticonvulsants (*e.g.,* primidone, zonisamide, levetiracetam, mesuximide, eslicarbazepine acetate, pregabalin), beta-2 antagonists (*e.g.,* propranolol, alprenolol, carteolol, nadolol, sotalol, timolol), CB agonists (*e.g.,* nabilone and WIN55,212-2 ((R)-(+)-[2,3-Dihydro-5-methyl-3-(4-morpholinylmethyl)pyrrolo[1,2,3-de]-1,4-benzoxazin-6-yl]-1-naphthalenylmethanone mesylate)), D2 agonists (*e.g.,* cabergoline bromocriptine, quinpirole, terguride, lisuride), D2/5-HT2A + antagonists (*e.g.* clozapine and quetiapine), DA agonists (*e.g.* pergolide, bromocriptine, quinpirole, apomorphine), dopamine agonists (*e.g.,* pramipexole, ropinirole, cabergoline, rotigotine), dopaminergic stabilizers (*e.g.* ACR325, aripiprazole, pridopidine), non-ergoline dopamine agonists *(e.g.,* ropinirole, pramipexole), dopaminergic function enhancers/glutamate release inhibitors (*e.g.,* safinamide), monoamine oxidase (MAO) inhibitors, including MAO-A, and MAO-B inhibitors (*e.g.,* rasagiline, selegiline, safinamide, EVT-302 (Evotec AG)), combinations of MAO-B inhibitors and acetylcholinesterase inhibitors (*e.g.,* ladostigil (TV-3326)), acetylcholinesterase inhibitors (*e.g.,* doepezil), a cholinesterase inhibitor that inhibits both butyrylcholinesterase and acetylcholinesterase *(e.g.,* rivastigmine), H2 antagonists *(e.g.,* famotidine, cimetidine, burimamide, ranitidine, metiamide), kynurenic hydroxylase inhibitors (*e.g.* Ro 61-8048 (3,4-Dimethoxy-N-[4-(3-nitrophenyl)-2-thiazolyl]benzene sulfonamide), FCE 28833 [(R,S)-2-amino-4-oxo-4-(3',4'-dichlorophenyl) butanoic acid]), L-type Ca2+ antagonists including L-type calcium channel blockers (*e.g.,* isradipine, amlodipine, azelnidipine, cilnidipine, lacidipine, nimodipine, nilvadipine), topiramate, mGluR5 antagonists (*e.g.,* AFQ056, 6-methyl-2-(phenylethynyl)pyridine), monoamine reuptake inhibitors (*e.g.,* NS2330 (tesofensine), bicifadine, DOV-102,677 [(5R)-5-(3,4-dichlorophenyl)bicyclo[3.1.0]hexane]), mu opiod receptor agonists, nicotine (*e.g.,* NP002), nicotinic agonists or partial agonists (*e.g.,* nicotine, acetylcholine, choline, epibatidine, lobeline, varenicline, sazetidine-A, DMXB-A (GTS-21, 3-2,4 dimethoxybenzylidene), SIB-1508Y (3-Ethynyl-5-[(2S)-1-methyl-2-pyrrolidinyl]pyridine)), nicotinic antagonists (*e.g.,* chlorisondamine, d-tubocurarine, mecamylamine, bupropion), nitric oxide synthase inhibitors (*e.g.,* 7-nitroindazole and NG-nitro-L-arginine), NMDA receptor antagonists (*e.g.,* remacemide, amantadine, memantine, rolicyclidine, ketamine, aptiganel), NMDA modulators (*e.g.,* Neu-120), NMDA/Sigma-1 antagonists *(e.g.,* dextromethorphan), nootropic compounds *(e.g.,* levitircetam), NR1A/2B NMDA antagonists (*e.g.,* Cl-1041 (5-[2-[4-(4-fluorobenzyl)piperidin-1-yl]ethylsulfinyl]-2,3-dihydro-1H-benzimidazol-2-one)), NR2B selective NMDA antagonists (*e.g.,* MK0657, CP-101,606 (traxoprodil), eliprodil, Ro 25-6981 ((±)-(R*,S*)-α-(4-hydroxyphenyl)-β-methyl-4-(phenylmethyl)-1-piperidine propanol), EVT-101 (Evotec AG), EVT-103 (Evotec AG)), omega-3 fatty acids (*e.g.,* docosahexaenoic acid), opiate antagonists *(e.g.,* naltrexone), anticholinergics *(e.g.,* amantadine, ipratropium bromide, oxitropium bromide, tiotropium), selective kappa opioid agonists (*e.g.,* TRK820 (17-cyclopropylmetbyl-3,14β-dihydroxy-4,5α-epoxy-6β-[*N*-methyl-*trans*-3-(3-furyl) acrylamido]morphinan hydrochloride) and U50,488 (2-(3,4-dichlorophenyl)-N-methyl-N-[(1R,2R)-2-pyrrolidin-1-ylcyclohexyl]acetamide)), SSRIs (*e.g.,* fluoxetine, paroxetine, sertraline, escitalopram, citalopram, fluvoxamine), AP09004, KW6500 (apomorphine), 4-hydroxyphenylpyruvate dioxygenase (HPPD) inhibitors (*e.g.,* SYN-118, Synosia Therapeutics), and phosphodiesterase (PDE) inhibitors, including at least PDE4 inhibitors (*e.g*., ronomilast, roflumilast, mesembrine, rolipram, ibudilast, piclamilast, luteolin, cilomilast, tofimilast (9-cyclopentyl-5,6-dihydro-7-ethyl-3-(thien-2-yl)-9H-pyrazolo(3,4-c)-1,2,4-triazolo(4,3-a)pyridine), tipelukast, ibudilast, apremilast ((S)-N-[2-[1-(3-ethoxy-4-methoxyphenyl)-2-methanesulfonylethyl]-1,3-dioxo-2,3-dihydro-1H-isoindol-4-yl] acetamide, Celgene Corporation), CC-1088 (a thalidomide analog inhibitor of PDE4, Celgene Corporation), 8-biarylnaphthyridinones such as MK-0952 (Merck), DC-AT-46 (7-benzylamino-6-chloro-2 piperazino-4-pyrrolidino-pteridine), HT-0712 ((3R,5R)-5-(3-(cyclopentyloxy)-4-methoxyphenyl)-3-(3-methylbenzyl)piperidin-2-one, Helicon Therapeutics, Inc.), MEM-1414 (Roche), MEM-1917 (Roche), MEM-1018 (Roche), MEM-1091 (Roche), DG071 (decode Genetics), and PDE2 inhibitors (*e.g.,* BAY 60-7550).

In one embodiment, eltoprazine or a pharmaceutically acceptable acid addition salt thereof is administered optionally in combination with one or more additional compound(s) listed above for prevention, attenuation, and/or treatment of dyskinesia or other motor disorder side effect that is associated with L-DOPA therapy or other dopamine-related drugs in Parkinson's patients. In another embodiment, eltoprazine or a pharmaceutically acceptable acid addition salt thereof is administered optionally in combination with one or more additional compound(s) listed above for prevention, attenuation, and/or treatment of PD.

In yet another embodiment, L-DOPA is administered to a human adult PD patient in need thereof, and following this administration, by the methods described herein, eltoprazine or a pharmaceutically acceptable acid addition salt thereof is administered optionally in combination with one or more additional compound(s) listed above for prevention, attenuation, and/or treatment of dyskinesia that is associated with L-DOPA therapy. In yet another embodiment, L-DOPA is administered to a human adult PD patient in need thereof following the administration of eltoprazine or a pharmaceutically acceptable acid addition salt thereof optionally in combination with one or more additional compound(s) listed above for prevention, attenuation, and/or treatment of dyskinesia that is associated with L-DOPA therapy.

In yet another embodiment, L-DOPA is administered to a human adult PD patient in need thereof, and following this administration, by the methods described herein, eltoprazine or a pharmaceutically acceptable acid addition salt thereof is administered after the development of motor side effects, optionally in combination with one or more additional compound(s) listed above for prevention, attenuation, and/or treatment of dyskinesia that is associated with L-DOPA therapy.

In yet another embodiment, L-DOPA is administered to a human adult PD patient in need thereof, and following this administration, by the methods described herein, eltoprazine or a pharmaceutically acceptable acid addition salt thereof is administered prior to the development of motor side effects, optionally in combination with one or more additional compound(s) listed above for prevention, attenuation, and/or treatment of dyskinesia that is associated with L-DOPA therapy.

In one embodiment, eltoprazine or a pharmaceutically acceptable acid addition salt thereof is administered in combination with at least one anticonvulsant. In another embodiment, eltoprazine or a pharmaceutically acceptable acid addition salt thereof is administered in combination with primidone. In yet another embodiment, eltoprazine or a pharmaceutically acceptable acid addition salt thereof is administered in combination with zonisamide.

In one embodiment, eltoprazine or a pharmaceutically acceptable acid addition salt thereof is administered in combination with at least one mGluR5 antagonist. In another embodiment, eltoprazine or a pharmaceutically acceptable acid addition salt thereof is administered in combination with AFQ056.

In one embodiment, eltoprazine or a pharmaceutically acceptable acid addition salt thereof is administered in combination with at least one L-type calcium channel blocker. In other embodiments, eltoprazine or a pharmaceutically acceptable acid addition salt thereof is administered in combination with isradipine, nimodipine, or nilvadipine.

In one embodiment, eltoprazine or a pharmaceutically acceptable acid addition salt thereof is administered in combination with at least one NMDA receptor antagonist. In another embodiment, eltoprazine or a pharmaceutically acceptable acid addition salt thereof is administered in combination with remacemide. In yet another embodiment, eltoprazine or a pharmaceutically acceptable acid addition salt thereof is administered in combination with amantadine.

In one embodiment, eltoprazine or a pharmaceutically acceptable acid addition salt thereof is administered in combination with at least one NMDA 2B receptor antagonist.

In another embodiment, eltoprazine or a pharmaceutically acceptable acid addition salt thereof is administered in combination with Ro 25-6981.

In yet another embodiment, eltoprazine or a pharmaceutically acceptable acid addition salt thereof is administered in combination with EVT-101 or EVT-103.

In one embodiment, eltoprazine or a pharmaceutically acceptable acid addition salt thereof is administered in combination with at least one adenosine A₂ₐ receptor antagonist. In another embodiment, eltoprazine or a pharmaceutically acceptable acid addition salt thereof is administered in combination with KW-6002 (istradefylline). In a further embodiment, eltoprazine or a pharmaceutically acceptable acid addition salt thereof is administered in combination with SCH420814 (preladenant).

In one embodiment, eltoprazine or a pharmaceutically acceptable acid addition salt thereof is administered in combination with at least one alpha-2 adrenergic receptor antagonist. In another embodiment, eltoprazine or a pharmaceutically acceptable acid addition salt thereof is administered in combination with JP1730 (fipamezole). In a further embodiment, eltoprazine or a pharmaceutically acceptable acid addition salt thereof is administered in combination with idazoxan. In yet another embodiment, eltoprazine or a pharmaceutically acceptable acid addition salt thereof is administered in combination with SYN-115 (4-Hydroxy-N-[4-methoxy-7-(4-morpholinyl)-2-benzothiazolyl]-4-methyl-1-piperidinecarboxamide, Synosia Therapeutics).

In one embodiment, eltoprazine or a pharmaceutically acceptable acid addition salt thereof is administered in combination with at least one 4-hydroxyphenylpyruvate dioxygenase (HPPD) inhibitor. In one embodiment, eltoprazine or a pharmaceutically acceptable acid addition salt thereof is administered in combination with SYN-118, Synosia Therapeutics).

In one embodiment, eltoprazine or a pharmaceutically acceptable acid addition salt thereof is administered in combination with at least one MAO-B inhibitor. In one embodiment, eltoprazine or a pharmaceutically acceptable acid addition salt thereof is administered in combination with safinamide, rasagiline, selegiline, or ladostigil.

In one embodiment, eltoprazine or a pharmaceutically acceptable acid addition salt thereof is administered in combination with at least one nicotinic acetylcholine receptor (nAChR) modulator. nAChR modulators include any compound that modulate the effect of the nAChR, including but not limited to nAChR selective or non-selective agonists, selective or non-selective partial agonists, competitive and non-competitive antagonists. In one embodiment, eltoprazine or a pharmaceutically acceptable acid addition salt thereof is administered in combination with nicotine, acetylcholine, choline, epibatidine, lobeline, varenicline, or sazetidine-A.

In one embodiment, eltoprazine or a pharmaceutically acceptable acid addition salt thereof is administered in combination with at least one phosphodiesterase (PDE) inhibitors, including at least PDE4 inhibitors and/or PDE2 inhibitors. In one embodiment, eltoprazine or a pharmaceutically acceptable acid addition salt thereof is administered in combination with ronomilast.

In one embodiment, the invention is not used for prevention, attenuation, and/or treatment of the associated cognitive impairment in PD. In another embodiment, the invention is not used for prevention, attenuation, and/or treatment of any one or more of the following: Alzheimer's disease, Huntington's disease, Cushing's syndrome, Lewy body disease, multiple sclerosis, stroke, addictive disorders, pervasive development disorder, Fragile X syndrome, anxiety disorders, Prader-Willi Syndrome, schizophrenia, bipolar disorder, depressive disorders, vascular dementia, mild cognitive impairment, dementia, or delirium.

In one embodiment, the methods of the invention encompass preventing, attenuating, and/or treating a motor side effect associated with a dopamine-related drug for the treatment of PD, which is not L-DOPA.

In some embodiments, the invention encompasses preventing, attenuating, and/or treating in human adult patients with PD, movement disorders, or motor disorder side effects associated with dopamine-related drugs, in addition to preventing, attenuating, and/or treating PD, by administering to a human adult patient in need thereof a therapeutic dose of eltoprazine, in combination with deep brain stimulation as performed in a surgical procedure, or in combination with magnetic brain stimulation, such as transcranial magnetic stimulation.

In one embodiment, the invention encompasses preventing, attenuating, and/or treating in human adult patients with PD, movement disorders, or motor disorder side effects associated with dopamine-related drugs, in addition to preventing, attenuating, and/or treating PD, by administering to a human adult patient in need thereof a therapeutic dose of eltoprazine in combination with deep brain stimulation and/or transcranial magnetic stimulation, and optionally in combination with any other compound described herein. Any order of treatment found to be beneficial is contemplated by the invention. For example, the deep brain stimulation and/or transcranial magnetic stimulation may precede the dopamine-related drug treatment, may follow the dopamine-related drug treatment, may precede the eltoprazine treatment, or may follow the eltoprazine treatment.

The doses of the compounds used in treating the disorders described herein in accordance with this invention will vary in the usual way with the seriousness of the disorder, the weight, and metabolic health of the individual in need of treatment. The preferred initial doses for the general patient population will be determined by routine dose-ranging studies, as are conducted, for example, during clinical trials. Therapeutically effective doses for individual patients may be determined, by titrating the amount of drug given to the individual to arrive at the desired therapeutic or prophylactic effect, while minimizing side effects.

Useful doses of eltoprazine are from 5 mg/day to 7.5 mg/dayln some embodiments, the daily dose of eltoprazine and/or related compounds is about 5 mg, 7 mg or 7.5 mg /day. Administration schedules may also be altered to achieve a therapeutically effective concentration of compound to treat the disorder or symptoms described herein.

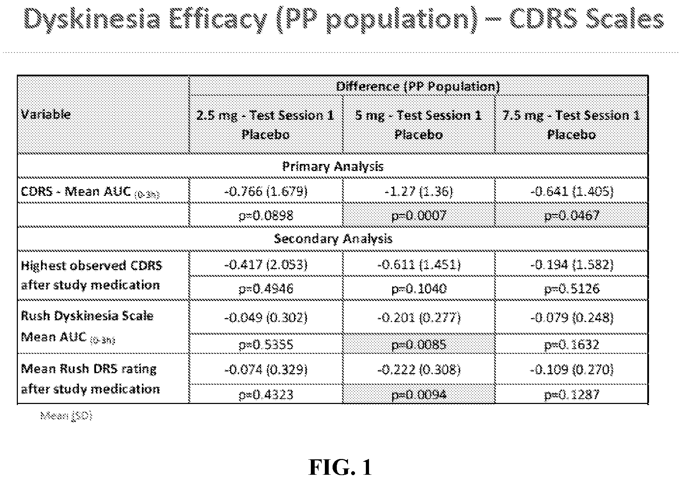

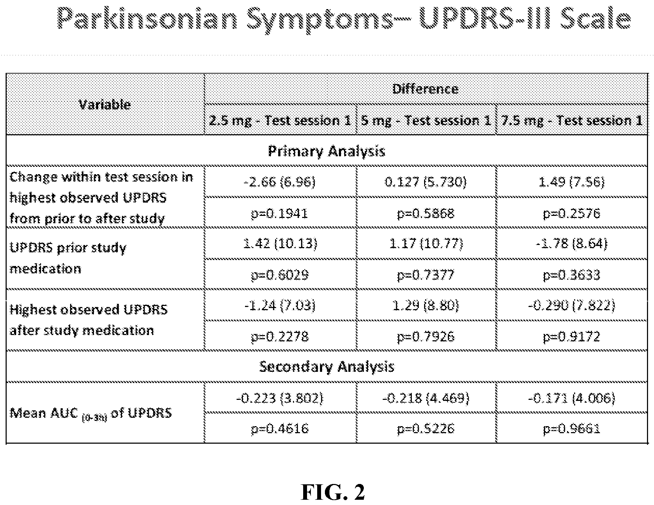

The inventors have discovered that particularly preferred and efficacious doses of eltoprazine for reducing and/or treating levodopa-induced dyskinesia in human adult Parkinson's patients are 5 mg/day and 7.5 mg/day, as shown in Example 1, and Figure 1. In addition, the inventors have shown that administration of eltoprazine to human adult Parkinson's patients already on levodopa treatment who then receive eltoprazine, do not show a reduction in the efficacy of levodopa therapy for treating PD, as shown in Example 1, and Figure 2. Further, eltoprazine was well-tolerated, and there were no serious adverse events from administration.

In some embodiments, eltoprazine and/or related compounds may be administered once per day, twice per day, thrice per day, 4 times per day, 5 times per day, 7 times per day or 10 times per day. In a preferred embodiment, eltoprazine is administered once per day. Often the dosage is divided equally throughout the day, however in some embodiments to treat certain disorders or symptoms, it may be useful to bias the dosage administration schedule so that most of the daily treatment is administered at the beginning half of the day. In some embodiments, about 50%, 60%, 70% or 80% of the dosage is administered in the first half of the day. In other embodiments, it may be more appropriate to administer most of the dosage in the latter half of the day so that about 50%, 60% , 70% or 80% of the dosage is administered in the latter half of the day.

Eltoprazine and, optionally, at least one additional compound, may be administered before, concurrently, or after administration of L-DOPA or other dopamine-related drug. In one embodiment, eltoprazine and, optionally, at least one additional compound, is administered before administration of L-DOPA or other dopamine-related drug. In one embodiment, after administration of eltoprazine and, optionally, at least one additional compound, the dose of L-DOPA or other dopamine-related drug is reduced.

Eltoprazine may be administered before, concurrently, or after administration of at least one additional compound. In one embodiment, eltoprazine is administered concurrently with one or more of the following additional compounds: anticonvulsants, mGluR5 antagonists, L-type calcium channel blockers, NMDA receptor antagonists, MAO-B inhibitors, HPPD inhibitors, adenosine A₂ₐ receptor antagonists and/or alpha-2 adrenergic receptor antagonists.

Eltoprazine and, optionally, at least one additional compound, may be administered before, concurrently, or after onset of symptoms. In one embodiment, the symptoms include a motor disorder side effect from use of L-DOPA or other dopamine-related drugs. In one embodiment, Eltoprazine agonist and, optionally, at least one additional compound, is/are administered after the development of a motor disorder side effect. In one embodiment, Eltoprazine and, optionally, at least one additional compound, is/are administered before the development of a motor disorder side effect.

Administration of the compounds of this invention may be by any method used for administering therapeutics, such as for example, oral, parenteral, intravenous, intramuscular, subcutaneous, rectal, or topical administration, such as through the use of a transdermal patch.

It will be appreciated by one of ordinary skill in the art that age of the human adult patient with the conditions described herein may respond to treatment at different degrees depending on factors such as dosage or administration or the presence of other factors or co-morbid conditions. Therefore, one of ordinary skill in the art will appreciate that the methods described herein may be directed to a particular age group.

In addition to comprising the therapeutic compounds for use in this invention, especially eltoprazine [1-(2,3-dihyro-1,4-benzodioxin-5-yl) piperazine] or pharmaceutically acceptable salts (preferably HCl in the case of eltoprazine) or pro-drug thereof, the pharmaceutical compositions for use with this invention may also comprise a pharmaceutically acceptable carrier. Such carriers may comprise additives, such as preservatives, excipients, fillers, wetting agents, binders, disintegrants, buffers may also be present in the compositions of the invention. Suitable additives may be, for example magnesium and calcium carbonates, carboxymethylcellulose, starches, sugars, gums, magnesium or calcium stearate, coloring or flavoring agents, and the like. There exists a wide variety of pharmaceutically acceptable additives for pharmaceutical dosage forms, and selection of appropriate additives is a routine matter for those skilled in art of pharmaceutical formulation.

The compositions may be in the form of tablets, capsules, powders, granules, lozenges, suppositories, reconstitutable powders, or liquid preparations such as oral or sterile parenteral solutions or suspensions.

In order to obtain consistency of administration it is preferred that a composition of the invention is in the form of a unit dose. Unit dose forms for oral administration may be tablets, capsules, and the like, and may contain conventional excipients such as binding agents, for example syrup, acacia, gelatin, sorbitol, tragacanth, or polyvinylpyrrolidone; and carriers or fillers, for example lactose, sugar, maize-starch, calcium phosphate, sorbitol or glycine. Additives may include disintegrants, for example starch, polyvinylpyrrolidone, sodium starch glycolate or microcrystalline cellulose; preservatives, and pharmaceutically acceptable wetting agents such as sodium lauryl sulphate.

In addition to unit dose forms, multi-dosage forms are also contemplated to be within the scope of the invention. Modified or controlled release dosage forms are contemplated for use in the invention, including, but not limited to sustained release dosage forms, extended release dosage forms, delayed release dosage forms, and pulsatile release dosage forms.

Suitable polymers for use in the controlled release formulations of the present invention include, but are not limited to uncrosslinked, linear polymers including cellulosic polymers, preferably hydroxyethyl cellulose, sodium carboxymethyl cellulose, hydroxypropylmethyl cellulose and hydroxypropyl cellulose, microcrystalline cellulose, methyl cellulose, and ethyl cellulose, and combinations thereof; covalently crosslinked insoluble polymers such as high molecular weight crosslinked homopolymers and copolymers of (meth) acrylic acid including carbopol resins, or mixtures of these uncrosslinked and covalently crosslinked polymers. Additionally suitable polymers include acrylic acid, methacrylic acid, methyl acrylate, ammonio methylacrylate, ethyl acrylate, methyl methacrylate and/or ethyl methacrylate, vinyl polymers and copolymers such as polyvinyl pyrrolidone, polyvinyl acetate, polyvinylacetate phthalate, vinylacetate crotonic acid copolymer, and ethylene-vinyl acetate copolymers, to name a few. Various combinations of two or more of the above polymers are also contemplated for use in the dosage forms of the invention.

Delayed release compositions may be prepared, for example, by employing slow release coatings, micro encapsulation, and/or slowly dissolving polymers.

The solid oral compositions may be prepared by conventional methods of blending, filling, tabletting or the like. Repeated blending operations may be used to distribute the active agent throughout those compositions employing large quantities of fillers. Such operations are conventional in the art. The tablets may be coated according to methods well known in normal pharmaceutical practice, for example with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol syrup, methyl cellulose, gelatin, hydroxyethylcellulose, carboxymethylcellulose, aluminum stearate gel, and hydrogenated edible fats; emulsifying agents, for example lecithin, sorbitan monooleate, or acacia; non-aqueous vehicles (which may include edible oils), for example almond oil or fractionated coconut oil, oily esters such as esters of glycerine, propylene glycol, or ethyl alcohol; preservatives, for example methyl or propyl p-hydroxybenzoate or sorbic acid; and if desired conventional flavoring or coloring agents.

For parenteral administration, fluid unit dosage forms are prepared utilizing the compound and a sterile vehicle, and, depending on the concentration used, can be either suspended or dissolved in the vehicle. In preparing solutions, the compound can be dissolved in water or saline for injection and filter sterilized before filling into a suitable vial or ampoule and sealing. Advantageously, additives such as a local anesthetic, preservative and buffering agent can be dissolved in the vehicle. Suitable buffering agents are, for example, phosphate and citrate salts. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by conventional means, for example by exposure to radiation or ethylene oxide, before being suspended in the sterile vehicle. Advantageously, a surfactant or wetting agent is included in the composition to facilitate uniform distribution of the compound.

Eltoprazine and, optionally, at least one additional compound may be provided in a kit. In one embodiment, a kit may comprise eltoprazine, and at least one additional compound. In one embodiment, a kit may comprise eltoprazine, L-DOPA or other dopamine-related drug, and optionally at least one additional compound. In one embodiment, a kit may comprise eltoprazine, L-DOPA, and amantadine. In another embodiment, a kit may comprise eltoprazine, L-DOPA, and KW-6002 (istradefylline). In a further embodiment, a kit as in any one of the previously described may also include instructions for administration of the compounds. In another embodiment, a kit may comprise eltoprazine, at least one dopamine precursor, at least one DDCI, and instructions for administering the compounds. In another embodiment, a kit may comprise eltoprazine, at least one dopamine precursor, at least one DDCI, at least one COMT inhibitor, and instructions for administering the compounds.

Certain modifications and improvements will occur to those skilled in the art upon a reading of the foregoing description. It should be understood that all such modifications and improvements have been deleted herein for the sake of conciseness and readability but are properly within the scope of the following claims. It is understood that the following examples and embodiments described herein are for illustrative purposes only and that various modifications or changes in light thereof will be suggestive to persons skilled in the art and are to be included within the spirit and purview of this application and the scope of the appended claims.

### EXAMPLE 1

### Treatment of motor disorder side effects associated with L-DOPA therapy using eltoprazine in human patients.

This example describes a multicenter, randomized, double-blind, placebo-controlled, dose finding study of oral eltoprazine in Parkinson's patients with L-DOPA induced dyskinesias, in a levodopa challenge-dose setting in Parkinson's Disease. A total of 22 patients participated in the study, out of which 18 patients fulfilled the protocol in terms of eligibility, interventions, and outcome assessment.

### Inclusion Criteria:

Subjects had to meet all of the following criteria to be eligible for the study:
1. Each patient had Parkinson's disease, defined according to the UK Brain Bank Criteria (see Hughes et al., J Neurol Neurosurg Psychiatry, 1992. 55(3): p. 181-4; Lees et al., Lancet, 2009. 373: p. 2055-66). Bradykinesia symptoms were combined with one of resting tremor, rigidity or postural imbalance.
2. Each patient had been treated for at least 3 years with L-DOPA prior to this study.
3. Each patient had significant dyskinesias after L-DOPA dosages according to clinical experience.
4. Each patient had significant dyskinesias after the administration of a single challenge dose of L-DOPA using two dyskinesia rating scales. The screening test was performed with a challenge dose of 150% of the normal regular dose of L-DOPA. If no dyskinesias was present during the first challenge, the challenge was repeated on an alternate day (and) significant dyskinesias in a patient self-administered diary with 3 levels with 3 grades ("off," "on without dyskinesias," "on with hyperkinesias") after the challenge dose has been given. Patients were included if one of the two challenge tests were positive and diary was positive for dyskinesias. In addition to the observed types of dyskinesias (dystonia, hyperkinesias), a temporal patterns were described as "peak-of dose," "end-of-dose dyskinesias" and "bi-phasic dyskinesias." Quantification was made by "peak-of-dose" ratings and area-under-the-curve (AuC) of scores.
5. Each patient was over 18 years of age.
6. This inclusion criterion applied to females of child-bearing potential (not surgically sterilized and between menarche and 1 year postmenopausal) only. Each female patient tested negative for pregnancy at the time of enrollment based on a serum pregnancy test and agreed to use a reliable method of birth control during the study.
7. Each patient signed informed consent.
8. Each patient had to able to communicate effectively with the investigator and study coordinator.

### Exclusion Criteria:

The presence of any of the following conditions excluded a subject from the study:
1. Exclusion criteria for Parkinson's disease according to the UK Brain Bank Criteria for Parkinson's disease.
2. Fulfillment of any other atypical parkinsonism diagnosis according to published criteria for multiple system atrophy (see Gilman, S., et al., Neurology, 2008. 71(9): p. 670-6), progressive supranucelar paresis (see Litvan, I., et al., Neurology, 1996. 47(1): p. 1-9), dementia with Lewy body (see McKeith, I.G., et al., Neurology, 2005. 65(12): p. 1863-72), corticobasal gangliotic disease (see Litvan, I., et al., J Neuropathol Exp Neurol, 1996. 55(1): p. 97-105), and dementia with Parkinson's disease (see Emre, M., et al., Mov Disord, 2007. 22(12): p. 1689-707; quiz 1837).
3. Any suspected secondary parkinsonism; drug induced parkinsonism; toxininduced parkinsonism; trauma-induced parkinsonism; normal pressure hydrocephalus and vascular parkinsonism.
4. Ongoing treatment with any selective serotonin re-uptake inhibitors (SSRI) or any combined serotonin-norepinephrine re-uptake inhibitors (SNRI) 4 weeks prior to the study.
5. Ongoing treatment with anti-parkinsonism medications (Cabergoline; Duodopa infusion; ApoGo infusion; Amantadine; Memantine if used against dyskinesias), and other medication with the potential for drug-interactions.
6. Significant depression defined as > 18 in the Montgomery Åsberg Depression Rating Scale combined with a clinical evaluation as to any clinical relevant depression.
7. Pregnant or breast-feeding.
8. Reduced kidney function; defined as a creatinine level > 120 µmol/L.
9. Reduced liver function, defined as aspartate aminotransferase, ASAT > 1.0 µkat/L or alanine aminotransferase, ALAT > 1.0 µkat/L or glutamyl transpeptidase, GT > 1.6 µkat/L, or total bilirubin > 30 µmol/L, or alkaline phosphatase, ALP > 6.0 µkat/L.
10. History of any other medical condition thought to interfere with the study or study medication (*i.e.,* recent myocardial infarction, uncontrolled diabetes, uncontrolled hypertension (systolic blood pressure > 180 mmHg), ongoing severe infection).
11. Receipt of an investigational drug within 30 days or 5 half-lives of the drug, whichever is longer, prior to entering this study.
12. Any known allergy to eltoprazine or the constituents of the study medication and the placebo capsules.
13. Any indication that patients are unsuitable in any other way to participate in this study, in the opinion of the investigator.

This study also assessed the safety and tolerability of eltoprazine in adults with Parkinson's Disease using the following measures: a) population mean values for the change in dyskinesia ratings between the placebo and screening baseline values and any of the eltoprazine dosages used, calculated as the peak-effect on CDRS of any study medication; b) population mean values for the change in dyskinesia ratings between the placebo and screening baseline values and any of the eltoprazine dosages used, calculated as the AuC on Rush DRS of any study medication; c) any changes in the UPDRS-III total score; d) any change in the diary data set, between the baseline and placebo and any of the three study medication tests; e) any deterioration of the HADS scores after study medication compared with placebo; f) any development of depression over the course of the study period, determined by the Montgomery Åsberg Depression Rating Scale (MADRS) and clinical judgment; g) comparison between the effects on Rush Dyskinesia Rating Scale (DRS) and Clinical Dyskinesia Rating Scale (CDRS) Area under the Curve (AuC) and peak of dose effects for the three study medication dosages.

The study consisted of 7 visits, described below: a screening visit, five treatment visits, and one end-of-study visit.

### Screening Period (Visit 1)

During the first visit, patients underwent screening for inclusion/exclusion criteria and safety assessments. Symptoms of parkinsonism, depression, and anxiety were assessed, and screening for significant L-DOPA dyskinesias were conducted using a challenge dose (150%) of L-DOPA. Subjects who were taking a prohibited medication were required to complete a washout period of appropriate length, as determined by the investigator. All patients received challenge doses (150% - up to a maximum of 250 mg) of L-DOPA at screening and at each treatment visit. L-DOPA was administered as Sinemet (L-DOPA combined with carbidopa in a fixed ratio of 4:1, unless a patient has a known allergy or intolerance to this drug). If a patient is intolerant to Sinemet, an equivalent dose of Madopar Quick could be used. There was an observation period of 3 hours (6 x 30 min, or 180 minutes) after dosing.

### Double-Blind Treatment Period (Visits 2-6)

During each of five visits, and after a two-hour fast, each patient received a challenge dosage (150% - up to a maximum of 250 mg) of levodopa. Additionally, during each of the five treatment visits, patients were also treated with single dose treatments of oral capsules of three active study medication dosages (2.5 mg, 5 mg, or 7.5 mg of eltoprazine) or two placebo doses. The patients were periodically recorded for 180 minutes after treatment, and the videos were evaluated in a blinded manner.

Visit 2 occurred within 30 days of visit 1, and visits 3-6 followed one week apart from each other.

### Final Visit or Early Termination Visit (Visit 7)

During the final visit or early termination visit, symptoms of parkinsonism, depression, anxiety, and L-DOPA dyskinesias were assessed, to capture any eltoprazine-related treatment effects on the degree of parkinsonism and any change in mood-related symptoms. Safety assessments were also conducted. A patient diary completed prior to and after the study period was used to evaluate any changes in perceived dyskinesias by the patients. A two-three day diary with 3 symptom lines - "off," "on (normal)," "on with dyskinesias" were filled out by each patient between the screening and enrollment visits and in between visits 2 through 6 to evaluate any changes in perceived dyskinesia by the patients.

The final visit, 7, was scheduled 4 days after visit 6. All study visits occurred within a ±5-day window of the time points noted above.

After screening, each patient participated in the study for approximately 6-10 weeks. The duration of the study was about 30 weeks. Safety evaluations were based on reports of adverse effects, concomitant therapy, clinical laboratory results, medical history, physical examination, and vital signs. Patient videos were used to assess efficacy. Rating scales include UPDRS, CDRS, and Rush DRS. In addition, patient diaries were used for self-assessment of dyskinesia.

### Rating Scales

The term "Unified Parkinson's Disease Rating Scale - III" and "UPDRS-III" refer to a standardized tool used to measure Parkinson's Disease severity, as described by Fahn et al., in Recent Developments in Parkinson's Disease, Fahn et al. (eds.) Plurham Park, N.J.: Macmillian Healthcare Information, 2:153-163,1987.

The term "Clinical Dyskinesia Rating Scale" and "CDRS" refer to a modified abnormal involuntary movement scale (AIMS) allowing for independent rating of limbs, trunk, head/neck and face rated during the UPDRS movements. This scale can simultaneously rate dystonia and dyskinesias, as described by Goetz et al., in Movement Disorders, Vol. 9, No. 4, 1994, 390-394.

The term "Rush Dyskinesia Rating Scale" and "Rush DRS" refer to an observer-based rating scale based on fixed movements. The numerical parts of the Rush DRS are recorded separately from the descriptive parts, as described by Goetz et al., in Movement Disorders, Vol. 9, No. 4, 1994, 390-394.

The term "Hospital Anxiety & Depression Scale" and "HADS" refer to rating scales commonly used by doctors to determine the levels of anxiety and depression that a patient is experiencing, as described by Zigmond et al., in Acta Psychiatrica Scandinavica 67 (6): 361-370.

The term "Montgomery- Åsberg Depression Rating Scale" and "MADRS" refer to an observer based ten-item diagnostic questionnaire used by psychiatrists to measure the severity of depressive episodes in patients with mood disorders, as described by Montgomery et al., in British Journal of Psychiatry 134 (4): 382-89.

Patients presenting clinically with motor disorder side effects associated with L-DOPA therapy, including dyskinesia, were evaluated using the United Parkinson's Disease Rating Scale III (Recent Developments in Parkinson's Disease, vol. 2, Fahn et al. editors, Macmillan Publishing Co. Inc, 1987), an art-recognized dyskinesia severity scale (Marconi et al., Mov Disord, 9:2-12, (1994)). Briefly, the dyskinesia severity scale rates abnormal movements from 0 (none) to 4 (severe with markedly impaired function) in six different parts of the body (face, neck, and trunk, and four limbs).

### Efficacy Assessments

### Filming

Filming occurred 30 minutes prior to, and every 30 minutes up to 180 minutes after a challenge test of L-DOPA and study medication. Approximately 5 minutes of video filming were performed each time. The "UPRDS movements" in the UPDRS scale was performed (arms in different positions, repeated pronations-supinations of the wrists, finger tapping of opposing fingers, opening and closing of the fists, foot stamping, raising from the chair, walking with a turn and a balance test). Each patient also performed tasks for the "Dyskinesia Rating Scales." The same sequences were repeated prior to any challenge dose, and after every 30 minutes up to 180 minutes after the intake of the medications, for a total of 7 sequences. Each sequence was rated by two independent blinded raters using UPDRS, CDRS and Rush DRS. Video taped records of each sequence were assessed by separate individual qualified raters at both of the two clinical centers.

### Patient diary

During screening, patients were asked to perform a self-administered rating of their dyskinesias by using a diary with 3 levels with 3 grades ("off," "on without hyperkinesias," and "on with hyperkinesias"). On the days following screening, the patients self rated their symptoms over three days. A two day diary with 3-symptom lines ("off," "on," "on with dyskinesias") was filled out by patients between the screening and enrollment visits and in between the Visits 2 through 6 (one day before dosing and one day after dosing).

### Efficacy Analyses

Analyses were done on both the intention-to-treat (ITT) and per protocol (PP) population, if applicable. The change between test sessions of the highest observed Unified Parkinson's Disease Rating Scale-III (UPDRS) within each session will be calculated between the randomised placebo test session and each active test session and will be analysed with Wilcoxon Signed Rank test on the ITT population.

The change in Mean AUC_{0-3 hours} of CDRS ratings will be analyzed between the randomised Placebo Test session and each active Test session with Wilcoxon Signed Rank test on the ITT population. Changes to all secondary variables between the randomized placebo test session and each active test session were analyzed using Wilcoxon Signed Rank test on both ITT- and PP-population.

### Results

Statistical analyses demonstrated that eltoprazine administered to Parkinson's disease patients with levodopa-induced dyskinesia (LID), statistically significantly reduced LID at both the 5 mg dose (p = 0.0007) and the 7.5 mg dose (p = 0.0467), as compared to placebo, when measured by the CDRS scale (see Fig. 1). In addition, eltoprazine administration at these doses did not affect L-DOPA efficacy, as measured by the UPDRS score (see Fig. 2); both the 5 mg and 7.5 mg doses were statistically significant as compared to placebo). At the 5 mg dose, eltoprazine reduced LID in PD patients statistically significantly - as measured by the Rush Scale AUC.

Furthermore, eltoprazine was well tolerated in this study, as there were no serious adverse events.

## Claims

1. Eltoprazine or a pharmaceutically acceptable acid addition salt thereof for use in a method of preventing, attenuating and/or treating a movement disorder or a motor disorder side effect associated with administration of levodopa or a dopamine-related drug to a human adult patient having Parkinson's disease; the method comprising administering to said human adult patient having Parkinson's disease in need of treatment eltoprazine or a pharmaceutically acceptable acid addition salt thereof at a dose range of 5 mg/day to 7.5 mg/day.

2. Eltoprazine or a pharmaceutically acceptable acid addition salt thereof for use in the method according to claim 1, wherein said movement disorder or motor disorder side effect is dyskinesia, hyperkinesia or levodopa-induced dyskinesia (LID).

3. Eltoprazine or a pharmaceutically acceptable acid addition salt thereof for use in a method of preventing, attenuating and/or treating levodopa-induced dyskinesia (LID), the method comprising administering to a human adult patient having Parkinson's disease in need of treatment eltoprazine or a pharmaceutically acceptable acid addition salt thereof at a dose range of 5 mg/day to 7.5 mg/day.

4. Eltoprazine or a pharmaceutically acceptable acid addition salt thereof, for use in a method according to any one of claims 1 to 3, the method comprising administering to said patient a dose of levodopa and a dose of eltoprazine or a pharmaceutically acceptable acid addition salt thereof, wherein said eltoprazine is administered at a dose range of 5 mg/day to 7.5 mg/day.

5. Eltoprazine or a pharmaceutically acceptable acid addition salt thereof for use in the method according to claim 4, wherein said eltoprazine and said levodopa are concurrently active in said human.

6. Eltoprazine or a pharmaceutically acceptable acid addition salt thereof for use in the method according to claim 4 or claim 5, wherein said eltoprazine is administered to the human before said human develops dyskinesia associated with said levodopa administration.

7. Eltoprazine or a pharmaceutically acceptable acid addition salt thereof for use in the method according to claim 4 or claim 5, wherein said eltoprazine is administered to said human after said human develops dyskinesia associated with said levodopa administration.

8. Eltoprazine or a pharmaceutically acceptable acid addition salt thereof for use in the method according to any one of claims 4 to 7, wherein said eltoprazine is administered to the human before the administration of said levodopa.

9. Eltoprazine or a pharmaceutically acceptable acid addition salt thereof for use in the method according to any one of claims 1 to 8, wherein said eltoprazine is administered in combination with at least one additional active ingredient, wherein the at least one additional active ingredient is selected from the group consisting of:
an (mTOR) complex 1 (mTORCl) inhibitor;
a 5-HTla/2a/alpha-2 antagonist;
a 5-HT2a inverse agonist;
an adenosine A2a receptor antagonist;
an alpha-1 adrenoceptor antagonist;
an alpha-2 adrenergic receptor antagonist;
an AMPA antagonist;
an AMPA/kainate antagonist;
an anticonvulsant;
a beta-2 antagonist;
a CB agonist;
a D2/5-HT2A+ antagonist;
a dopaminergic stabilizer;
a dopaminergic function enhancer/glutamate release inhibitor;
a combinations of a MAO-B inhibitor and an acetylcholinesterase inhibitor;
an acetylcholinesterase inhibitor;
a cholinesterase inhibitor that inhibits both butyrylcholinesterase and
acetylcholinesterase;
an H2 antagonist;
a kynurenic hydroxylase inhibitor;
an L-type Ca2+ antagonist or L-type calcium channel blocker;
an mGluR5 antagonist;
a monoamine reuptake inhibitor;
a mu opioid receptor agonist
a nicotinic acetylcholine receptor (nAChR) modulator;
a nitric oxide synthase inhibitor;
a nootropic compound;
an NR1A/2B NMDA antagonist;
an NR2B selective NMDA antagonist;
an omega-3 fatty acid;
an opiate antagonist;
an anticholinergic;
an SSRI (Selective Serotonin Reuptake Inhibitor);
a 4-hydroxyphenylpyruvate dioxygenase (HPPD) inhibitor;
a phosphodiesterase (PDE) inhibitor such as a phosphodiesterase 4 (PDE4) inhibitor or a phosphodiesterase 2 (PDE2) inhibitor; and
carbidopa.

10. Eltoprazine or a pharmaceutically acceptable acid addition salt thereof for use in the method according to claim 9,
wherein the (mTOR) complex 1 (mTORCl) inhibitor is rapamycin;
wherein the 5-HTla/2a/alpha-2 antagonist is mirtazapine;
wherein the 5-HT2a inverse agonist is selected from ACP103 (pimavanserin), nelotanserin, eplivanserin, or AC-90179 [2-(4-Methoxyphenyl)-N-(4-methyl-benzyl)-N-(1-methyl-piperidin-4-yl)-acetamide Hydrochloride]);
wherein the adenosine A2a receptor antagonist is selected from istradefylline (KW-6002), SYN-115 (4-Hydroxy-N-[4-methoxy-7-(4-morpholinyl)-2-benzothiazolyl]-4-methyl-1-piperidinecarboxamide, (Synosia Therapeutics), preladenant (SCH420814), ZM-241,385 (4-(2-[7-amino-2-(2-furyl) [1,2,4]-triazolo[2,3-a][1,3,5]triazin- 5-yl amino]ethyl) phenol), VER-7835 (2-amino-6-(furan-2-yl)-N-(thiophen-2-ylmethyl)-9H-purine-9- carboxamide), MSX-3 (3-(3-hydroxypropyl)-7-methyl-8-(m-methoxystyryl)-1-propargylxanthine), or ST-1535 (2-butyl-9-methyl-8-(2H-1,2,3-triazol 2-yl)-9 H-purin-6-ylamine));
wherein the alpha-1 adrenoceptor antagonist is selected from HEAT (2-[[beta-(-4-hydroxyphenyl)ethyl]aminomethyl]-1-tetralone), alfuzosin, doxazosin, moxisylyte, prazosin, or trimazosin;
wherein the alpha-2 adrenergic receptor antagonist is selected from idazoxan, fipamezole (JP1730), atipamezole, mianserin, phentolamine, or tolazoline;
wherein the AMPA antagonist is E2007 (perampanel);
wherein the AMPA/kainate antagonist is LY300164 [7-acetyl-5-(4-aminophenyl)-8,9-dihydro-8-methyl-7H-1,3-dioxolo(4,5H)-2,3-benzodiazepine];
wherein the anticonvulsant is selected from primidone, zonisamide, levetiracetam, mesuximide, eslicarbazepine acetate, or pregabalin;
wherein the beta-2 antagonist is selected from propranolol, alprenolol, carteolol, nadolol, sotalol, or timolol;
wherein the CB agonist is selected from nabilone or WIN55,212-2 ((R)-(+)-[2,3-Dihydro-5-methyl-3-(4-morpholinylmethyl)pyrrolo[1,2,3-de]-1,4-benzoxazin-6-yl]-1-naphthalenylmethanone mesylate);
wherein the D2/5-HT2A+ antagonist is clozapine or quetiapine;
wherein the dopaminergic stabilizer is selected from ACR325, aripiprazole, or pridopidine;
wherein the dopaminergic function enhancer/glutamate release inhibitor is safinamide;
wherein the combination of the MAO-B inhibitor and acetylcholinesterase inhibitor is selected from rasagiline, selegiline, safinamide, or EVT-302 (Evotec AG) in combination with ladostigil (TV-3326);
wherein the acetylcholinesterase inhibitor is doepezil;
wherein the cholinesterase inhibitor that inhibits both butyrylcholinesterase and acetylcholinesterase is rivastigmine;
wherein the H2 antagonist is selected from famotidine, cimetidine, burimamide, ranitidine, or metiamide;
wherein the kynurenic hydroxylase inhibitor is selected from Ro 61-8048 (3,4-Dimethoxy-N-[4-(3-nitrophenyl)-2-thiazolyl]benzene sulfonamide or FCE 28833 [(R,S)-2-amino-4-oxo-4-(3',4'-dichlorophenyl) butanoic acid];
wherein the L-type Ca2+ antagonist or L-type calcium channel blocker is selected from isradipine, amlodipine, azelnidipine, cilnidipine, lacidipine, nimodipine, nilvadipine or topiramate;
wherein the mGluR5 antagonist is AFQ056 (6-methyl-2-(phenylethynyl)pyridine);
wherein the monoamine reuptake inhibitor is selected from NS2330 (tesofensine), bicifadine, or DOV-102,677 [(5R)-5-(3,4-dichlorophenyl)bicyclo[3.1.0]hexane];
wherein the nicotinic acetylcholine receptor (nAChR) modulator is selected from an nAChR selective or non-selective agonist; an nAChR selective or non-selective partial agonist; an nAChR competitive antagonist; an nAChR non-competitive antagonist; nicotine (NP002); acetylcholine; choline; epibatidine; lobeline; varenicline; sazetidine-A; DMXB-A (GTS-21, 3-2,4 dimethoxybenzylidene); or SIB-1508Y (3-Ethynyl-5-[(2S)-1-methyl-2-pyrrolidinyl]pyridine);
wherein the nicotinic antagonist is selected from chlorisondamine, d-tubocurarine, mecamylamine, or bupropion;
wherein the nitric oxide synthase inhibitor is selected from 7-nitroindazole or NG-nitro -L-arginine;
wherein the nootropic compound is levitircetam;
wherein the NR1A/2B NMDA antagonist is Cl-1041 (5-[2-[4-(4-fluorobenzyl)piperidin-1-yl]ethylsulfinyl]-2,3-dihydro-1H-benzimidazol-2-one));
wherein the NR2B selective NMDA antagonist is selected from MK0657, CP-101,606 (traxoprodil), eliprodil, Ro 25-6981 ((±)-(R*,S*)-α-(4-hydroxyphenyl)-β-methyl-4-(phenylmethyl)-1-piperidine propanol), EVT-101 (Evotec AG), or EVT-103 (Evotec wherein the omega-3 fatty acid is docosahexaenoic acid;
wherein the opiate antagonist is naltrexone;
wherein the anticholinergic is selected from amantadine, ipratropium bromide, oxitropium bromide, or tiotropium;
wherein the SSRI is selected from fluoxetine, paroxetine, sertraline, escitalopram, citalopram, or fluvoxamine;
wherein the 4-hydroxyphenylpyruvate dioxygenase (HPPD) inhibitor is SYN-118, (Synosia Therapeutics);
wherein the PDE4 inhibitor is selected from ronomilast, roflumilast, mesembrine, rolipram, ibudilast, piclamilast, luteolin, cilomilast, tofimilast (9-cyclopentyl-5,6-dihydro-7-ethyl-3-(thien-2-yl)-9H-pyrazolo(3,4-c)-1,2,4-triazolo(4,3-a)pyridine), tipelukast, ibudilast, apremilast ((S)-N-[2-[1-(3-ethoxy-4-methoxyphenyl)-2-methanesulfonylethyl]-1,3-dioxo-2,3-dihydro-1H-isoindol-4-yl] acetamide, Celgene Corporation), CC-1088 (a thalidomide analog inhibitor of PDE4, Celgene Corporation), 8-biarylnaphthyridinones such as MK-0952 (Merck), DC-AT-46 (7-benzylamino-6-chloro-2 piperazino-4-pyrrolidino-pteridine), HT-0712 ((3R,5R)-5-(3-(cyclopentyloxy)-4-methoxyphenyl)-3-(3-methylbenzyl)piperidin-2-one, Helicon Therapeutics, Inc.), MEM-1414 (Roche), MEM-1917 (Roche), MEM-1018 (Roche), MEM-1091 (Roche), or DG071 (decode Genetics); and
wherein the PDE2 inhibitor is BAY 60-7550.

11. Eltoprazine or a pharmaceutically acceptable acid addition salt thereof for use in the method according to claim 9 or claim 10, wherein said eltoprazine or a pharmaceutically acceptable acid addition salt thereof is administered with at least one anticonvulsant, which is, optionally, selected from primidone or zonisamide.

12. Eltoprazine or a pharmaceutically acceptable acid addition salt thereof for use in the method according to claim 9 or claim 10, wherein said eltoprazine or a pharmaceutically acceptable acid addition salt thereof is administered with at least one mGluR5 antagonist, which is, optionally, AFQ056.

13. Eltoprazine or a pharmaceutically acceptable acid addition salt thereof for use in the method according to claim 9 or claim 10, wherein said eltoprazine or a pharmaceutically acceptable acid addition salt thereof is administered with at least one L-type calcium channel blocker, which is, optionally, selected from isradipine, nimodipine, or nilvadipine.

14. Eltoprazine or a pharmaceutically acceptable acid addition salt thereof for use in the method according to claim 9 or claim 10, wherein said eltoprazine or a pharmaceutically acceptable acid addition salt thereof is administered with at least one NR2B selective NMDA antagonist, which is, optionally, selected from Ro 25-6981, EVT-101, or EVT-103.

15. Eltoprazine or a pharmaceutically acceptable acid addition salt thereof for use in the method according to claim 9 or claim 10, wherein said eltoprazine or a pharmaceutically acceptable acid addition salt thereof is administered with (i) at least one adenosine A2a receptor antagonist, which is, optionally, selected from istradefylline (KW-6002), SYN-115 (4-Hydroxy-N-[4-methoxy-7-(4-morpholinyl)-2-benzothiazolyl]-4-methyl-1-piperidinecarboxamide, or preladenant (SCH420814); or (ii) at least one alpha-2 adrenergic receptor antagonist, which is, optionally, selected from fipamezole (JP1730) or idazoxan.

16. Eltoprazine or a pharmaceutically acceptable acid addition salt thereof for use in the method according to claim 9 or claim 10, wherein said eltoprazine or a pharmaceutically acceptable acid addition salt thereof is administered with at least one 4-hydroxyphenylpyruvate dioxygenase (HPPD) inhibitor, which is, optionally, SYN-118.

17. Eltoprazine or a pharmaceutically acceptable acid addition salt thereof for use in the method according to claim 9 or claim 10, wherein said eltoprazine or a pharmaceutically acceptable acid addition salt thereof is administered with at least one nicotinic acetylcholine receptor (nAChR) modulator, which is, optionally, selected from an nAChR selective or non-selective agonist; an nAChR selective or non-selective partial agonist; an nAChR competitive antagonist; an nAChR non-competitive antagonist; nicotine (NP002); acetylcholine; choline; epibatidine; lobeline; varenicline; or sazetidine-A.

18. Eltoprazine or a pharmaceutically acceptable acid addition salt thereof for use in the method according to claim 9 or claim 10, wherein said eltoprazine or a pharmaceutically acceptable acid addition salt thereof is administered with at least one phosphodiesterase (PDE) inhibitor, which is, optionally, a phosphodiesterase 2 (PDE2) inhibitor or a phosphodiesterase 4 (PDE4) inhibitor.

19. Eltoprazine or a pharmaceutically acceptable acid addition salt thereof for use in the method according to any one of claims 9 to 18, wherein said eltoprazine or a pharmaceutically acceptable acid addition salt thereof and said at least one additional active ingredient are administered to said patient in an administration mode selected from:
(i) administration of a combination of said eltoprazine and said at least one additional active ingredient to said patient in need thereof before said patient develops a movement disorder, a motor disorder side effect associated with administration of levodopa or a dopamine-related drug to a human adult patient having Parkinson's disease; or
(ii) administration of a combination of said eltoprazine and said at least one additional active ingredient to said patient in need thereof after said patient develops a movement disorder, a motor disorder side effect associated with administration of levodopa or a dopamine-related drug to a human adult patient having Parkinson's disease.

20. Eltoprazine or a pharmaceutically acceptable acid addition salt thereof for use in the method according to any one of claims 9 to 19, wherein said eltoprazine is administered to the patient in a dosing regimen selected from:
(i) administration of said eltoprazine to the patient before said at least one additional active agent is administered;
(ii) administration of said eltoprazine to the patient after said at least one additional active agent is administered;
(iii) administration of said eltoprazine to the patient concurrently with the administration of said at least one additional active agent.

21. Eltoprazine or a pharmaceutically acceptable acid addition salt thereof according to claim 1 in combination with deep brain stimulation and/or transcranial magnetic stimulation.

22. Eltoprazine or a pharmaceutically acceptable acid addition salt thereof according to claim 21, wherein the order of said treatment comprises an administration regimen selected from:
(i) said deep brain stimulation and/or transcranial magnetic stimulation precedes treatment of the patient with levodopa or a dopamine-related drug;
(ii) said deep brain stimulation and/or transcranial magnetic stimulation follows treatment of the patient with levodopa or a dopamine-related drug ;
(iii) said deep brain stimulation and/or transcranial magnetic stimulation precedes said treatment of the patient with eltoprazine or a pharmaceutically acceptable acid addition salt thereof; or
(iv) said deep brain stimulation and/or transcranial magnetic stimulation follows said treatment of the patient with eltoprazine or a pharmaceutically acceptable acid addition salt thereof.

23. Eltoprazine or a pharmaceutically acceptable acid addition salt thereof according to any one of claims 1 to 22, wherein said dopamine-related drug is selected from a dopamine receptor agonist, which is, optionally, bromocriptine, pergolide, cabergoline, apomorphine, or lisuride; or a non-ergoline dopamine agonist, which is, optionally, ropinirole or pramipexole.

24. Eltoprazine or a pharmaceutically acceptable acid addition salt thereof, for use in a method according to any one of claims 1 to 8, the method comprising administering to said patient a dose of levodopa, a dose of carbidopa and a dose of eltoprazine or a pharmaceutically acceptable acid addition salt thereof.

25. Eltoprazine or a pharmaceutically acceptable acid addition salt thereof, for use in a method of preventing, attenuating and/or treating a motor disorder side effect associated with administration of levodopa or a dopamine-related drug to a human adult patient having Parkinson's disease, according to any one of claims 1 to 8, the method comprising administering to said patient a dose of levodopa, a dose of carbidopa, a dose of entacapone and a dose of eltoprazine or a pharmaceutically acceptable acid addition salt thereof.

26. Eltoprazine or a pharmaceutically acceptable acid addition salt thereof in combination with levodopa or a dopamine-related drug, for use in a method of preventing, attenuating and/or treating a human adult patient having Parkinson's disease; the method comprising administering to said human adult patient having Parkinson's disease in need of treatment levodopa or a dopamine-related drug and eltoprazine or a pharmaceutically acceptable acid addition salt thereof at a dose range of 5 mg/day to 7.5 mg/day.

27. Eltoprazine or a pharmaceutically acceptable acid addition salt thereof according to any one of claims 1 to 26, wherein said eltoprazine is administered at a dose of 5 mg/day.

28. Eltoprazine or a pharmaceutically acceptable acid addition salt thereof according to any one of claims 1 to 26, wherein said eltoprazine is administered at a dose of 7 mg/day.

29. Eltoprazine or a pharmaceutically acceptable acid addition salt thereof according to any one of claims 1 to 26, wherein said eltoprazine is administered at a dose of 7.5 mg/day.

## Patentansprüche

1. Eltoprazin oder ein pharmazeutisch verträgliches Säureadditionssalz davon, zur Verwendung in einem Verfahren zur Verhütung, Linderung und/oder Behandlung einer Bewegungsstörung- oder einer Motorikstörung-Nebenwirkung, die mit der Verabreichung von Levodopa oder einem Dopamin-verwandten Wirkstoff an einen menschlichen adulten Patienten mit Morbus Parkinson assoziiert ist; wobei das Verfahren Verabreichen, an den menschlichen adulten Patienten mit Morbus Parkinson, mit Behandlungsbedarf, von Eltoprazin oder einem pharmazeutisch verträglichen Säureadditionssalz davon in einem Dosisbereich von 5 mg/Tag bis 7,5 mg/Tag umfasst.

2. Eltoprazin oder ein pharmazeutisch verträgliches Säureadditionssalz davon, zur Verwendung im Verfahren nach Anspruch 1, wobei die Bewegungsstörung- oder Motorikstörung-Nebenwirkung Dyskinesie, Hyperkinese oder Levodopa-induzierte Dyskinesie (LID) ist.

3. Eltoprazin oder ein pharmazeutisch verträgliches Säureadditionssalz davon, zur Verwendung in einem Verfahren zur Verhütung, Linderung und/oder Behandlung von Levodopa-induzierter Dyskinesie (LID), wobei das Verfahren Verabreichen, an einen menschlichen adulten Patienten mit Morbus Parkinson, mit Behandlungsbedarf, von Eltoprazin oder einem pharmazeutisch verträglichen Säureadditionssalz davon in einem Dosisbereich von 5 mg/Tag bis 7,5 mg/Tag umfasst.

4. Eltoprazin oder ein pharmazeutisch verträgliches Säureadditionssalz davon, zur Verwendung in einem Verfahren nach einem der Ansprüche 1 bis 3, wobei das Verfahren Verabreichen, an den Patienten, einer Dosis Levodopa und einer Dosis Eltoprazin oder eines pharmazeutisch verträglichen Säureadditionssalzes davon umfasst, wobei das Eltoprazin in einem Dosisbereich von 5 mg/Tag bis 7,5 mg/Tag verabreicht wird.

5. Eltoprazin oder ein pharmazeutisch verträgliches Säureadditionssalz davon, zur Verwendung im Verfahren nach Anspruch 4, wobei das Eltoprazin und das Levodopa im Menschen gleichzeitig aktiv sind.

6. Eltoprazin oder ein pharmazeutisch verträgliches Säureadditionssalz davon, zur Verwendung im Verfahren nach Anspruch 4 oder Anspruch 5, wobei das Eltoprazin dem Menschen verabreicht wird, bevor der Mensch mit der Levodopa-Verabreichung assoziierte Dyskinesie entwickelt.

7. Eltoprazin oder ein pharmazeutisch verträgliches Säureadditionssalz davon, zur Verwendung im Verfahren nach Anspruch 4 oder Anspruch 5, wobei das Eltoprazin dem Menschen verabreicht wird, nachdem der Mensch mit der Levodopa-Verabreichung assoziierte Dyskinesie entwickelt hat.

8. Eltoprazin oder ein pharmazeutisch verträgliches Säureadditionssalz davon, zur Verwendung im Verfahren nach einem der Ansprüche 4 bis 7, wobei das Eltoprazin dem Menschen vor der Verabreichung des Levodopas verabreicht wird.

9. Eltoprazin oder ein pharmazeutisch verträgliches Säureadditionssalz davon, zur Verwendung im Verfahren nach einem der Ansprüche 1 bis 8, wobei das Eltoprazin in Kombination mit zumindest einem zusätzlichen aktiven Inhaltsstoff verabreicht wird, wobei der zumindest eine zusätzliche aktive Inhaltsstoff aus der Gruppe ausgewählt ist, die besteht aus:
einem (mTOR)-Komplex-1-(mTORC1-)Hemmer;
einem 5-HT1a/2a/Alpha-2-Antagonisten;
einem 5-HT2a-Inversagonisten;
einem Adenosin-A2a-Rezeptor-Antagonisten;
einem Alpha- 1-Adrenozeptor-Antagonisten;
einem Alpha-2-Adrenozeptor-Antagonisten;
einem AMPA-Antagonisten;
einem AMPA/Kainat-Antagonisten;
einem Antikonvulsivum;
einem Beta-2-Antagonisten;
einem CB-Agonisten;
einem D2/5-HT2A+ Antagonisten;
einem dopaminergen Stabilisierer;
einem Verstärker dopaminerger Funktion/Glutamatfreisetzungshemmer;
einer Kombination von einem MAO-B-Hemmer und einem Acetylcholinesterase-Hemmer;
einem Acetylcholinesterase-Hemmer;
einem Cholinesterase-Hemmer, der sowohl Butyrylcholinesterase als auch Acetylcholinesterase inhibiert;
einem H2-Antagonisten;
einem Kynurenin-Hydroxylase-Hemmer;
einem L-Typ-Ca²⁺-Antagonisten oder L-Typ-Kalziumkanal-Blocker;
einem mGluR5-Antagonisten;
einem Monoamin-Wiederaufnahme-Hemmer;
einem mu-Opioidrezeptor-Agonisten;
einem Nikotinischer-Acetylcholinrezeptor- (nAChR-) Modulator;
einem Stickoxid-Synthase-Hemmer;
einer nootropen Verbindung;
einem NR1A/2B-NMDA-Antagonisten;
einem NR2B-selektiven NMDA-Antagonisten;
einer Omega-3-Fettsäure;
einem Opiatantagonisten;
einem Anticholinergikum;
einem SSRI (selektiven Serotonin-Wiederaufnahme-Hemmer);
einem 4-Hydroxyphenylpyruvat-Dioxygenase- (HPPD-) Hemmer;
einem Phosphodiesterase- (PDE-) Hemmer wie einem Phosphodiesterase-4-(PDE4-) Hemmer oder einem Phosphodiesterase-2- (PDE2-) Hemmer; und
Carbidopa.

10. Eltoprazin oder ein pharmazeutisch verträgliches Säureadditionssalz davon, zur Verwendung im Verfahren nach Anspruch 9,
wobei der (mTOR)-Komplex-1-(mTORC1-) Hemmer Rapamycin ist;
wobei der 5-HT1a/2a/Alpha-2-Antagonist Mirtazapin ist;
wobei der 5-HT2a-Inversagonist aus ACP103 (Pimavanserin), Nelotanserin, Eplivanserin oder AC-90179 [2-(4-Methoxyphenyl)-N-(4-methyl-benzyl)-N-(1-methylpiperidin-4-yl)-acetamid-Hydrochlorid] ausgewählt ist;
wobei der Adenosin-A2a-Rezeptor-Antagonist aus Istradefyllin (KW-6002), SYN-115 (4-Hydroxy-N-[4-methoxy-7-(4-morpholinyl)-2-benzothiazolyl]-4-methyl-1-piperidincarboxamid (Synosia Therapeutics), Preladenant (SCH420814), ZM-241,385 (4-(2-[7-Amino-2-(2-furyl)-[1,2,4]-triazolo[2,3-a][1,3,5]triazin-5-ylamino]ethyl)phenol), VER-7835 (2-Amino-6-(furan-2-yl)-N-(thiophen-2-ylmethyl)-9H-purin-9-carboxamid), MSX-3 (3-(3-Hydroxypropyl)-7-methyl-8-(m-methoxystyryl)-1-propargylxanthin) oder ST-1535 (2-Butyl-9-methyl-8-(2H-1,2,3-triazol-2-yl)-9H-purin-6-ylamin) ausgewählt ist;
wobei der Alpha-1-Adrenozeptor-Antagonist aus HEAT (2-[[β-(-4-Hydroxyphenyl)ethyl]aminomethyl]-1-tetralon), Alfuzosin, Doxazosin, Moxisylyt, Prazosin oder Trimazosin ausgewählt ist;
wobei der Alpha-2-Adrenozeptor-Antagonist aus Idazoxan, Fipamezol (JP1730), Atipamezol, Mianserin, Phentolamin oder Tolazolin ausgewählt ist;
wobei der AMPA-Antagonist E2007 (Perampanel) ist;
wobei der AMPA/Kainat-Antagonist LY300164 [7-Acetyl-5-(4-aminophenyl)-8,9-dihydro-8-methyl-7H-1,3-dioxolo(4,5H)-2,3-benzodiazepin] ist;
wobei das Antikonvulsivum aus Primidon, Zonisamid, Levetiracetam, Mesuximid, Eslicarbazepinacetat oder Pregabalin ausgewählt ist;
wobei der Beta-2-Antagonist aus Propranolol, Alprenolol, Carteolol, Nadolol, Sotalol oder Timolol ausgewählt ist;
wobei der CB-Agonist aus Nabilon oder WIN55,212-2 ((R)-(+)-[2,3-Dihydro-5-methyl-3-(4-morpholinylmethyl)pyrrolo[1,2,3-de]-1,4-benzoxazin-6-yl]-1-naphthalinylmethanon-Mesylat) ausgewählt ist;
wobei der D2/5-HT2A+ Antagonist Clozapin oder Quetiapin ist;
wobei der dopaminerge Stabilisierer aus ACR325, Aripiprazol oder Pridopidin ausgewählt ist;
wobei der Verstärker dopaminerger Funktion/Glutamatfreisetzungshemmer Safinamid ist;
wobei die Kombination des MAO-B-Hemmers und des Acetylcholinesterase-Hemmers aus Rasagilin, Selegilin, Safinamid oder EVT-302 (Evotec AG) in Kombination mit Ladostigil (TV-3326) ausgewählt ist;
wobei der Acetylcholinesterase-Hemmer Donepezil ist;
wobei der Cholinesterase-Hemmer, der sowohl Butyrylcholinesterase als auch Acetylcholinesterase inhibiert, Rivastigmin ist;
wobei der H2-Antagonist aus Famotidin, Cimetidin, Burimamid, Ranitidin oder Metiamid ausgewählt ist;
wobei der Kynurenin-Hydroxylase-Hemmer aus Ro 61-8048 (3,4-Dimethoxy-N-[4-(3-nitrophenyl)-2-thiazolyl]benzolsulfonamid) oder FCE 28833 [(R,S)-2-Amino-4-oxo-4-(3',4'-dichlorphenyl)butansäure] ausgewählt ist;
wobei der L-Typ-Ca²⁺-Antagonist oder L-Typ-Kalziumkanal-Blocker aus Isradipin, Amlodipin, Azelnidipin, Cilnidipin, Lacidipin, Nimodipin, Nilvadipin oder Topiramat ausgewählt ist;
wobei der mGluR5-Antagonist AFQ056 (6-Methyl-2-(phenylethinyl)pyridin) ist; wobei der Monoamin-Wiederaufnahme-Hemmer aus NS2330 (Tesofensin), Bicifadin oder DOV-102,677 [(5R)-5-(3,4-Dichlorphenyl)bicyclo[3.1.0]hexan] ausgewählt ist;
wobei der Nikotinischer-Acetylcholinrezeptor- (nAChR-) Modulator aus einem selektiven oder nicht selektiven nAChR-Agonisten; einem selektiven oder nicht selektiven nAChR-Partialagonisten; einem kompetitiven nAChR-Antagonisten; einem nicht kompetitiven nAChR-Antagonisten; Nikotin (NP002); Acetylcholin; Cholin; Epibatidin; Lobelin; Vareniclin; Sazetidin-A; DMXB-A (GTS-21, 3-2,4-Dimethoxybenzyliden); oder SIB-1508Y (3-Ethinyl-5-[(2S)-1-methyl-2-pyrrolidinyl]pyridin) ausgewählt ist;
wobei der nikotinische Antagonist aus Chlorisondamin, d-Tubocurarin, Mecamylamin oder Bupropion ausgewählt ist;
wobei der Stickoxid-Synthase-Hemmer aus 7-Nitroindazol oder NG-Nitro-L-Arginin ausgewählt ist;
wobei die nootrope Verbindung Levitircetam ist;
wobei der NR1A/2B-NMDA-Antagonist Cl-1041 (5-[2-[4-(4-Fluorbenzyl)piperidin-1-yl]ethylsulfinyl]-2,3-dihydro-1H-benzimidazol-2-on) ist;
wobei der NR2B-selektive NMDA-Antagonist aus MK0657, CP-101,606 (Traxoprodil), Eliprodil, Ro 25-6981 ((±)-(R*,S*)-α-(4-Hydroxyphenyl)-β-methyl-4-(phenylmethyl)-l-piperidinpropanol), EVT-101 (Evotec AG) oder EVT-103 (Evotec) ausgewählt ist;
wobei die Omega-3-Fettsäure Docosahexaensäure ist;
wobei der Opiatantagonist Naltrexon ist;
wobei das Anticholinergikum aus Amantadin, Ipratropiumbromid, Oxitropiumbromid oder Tiotropium ausgewählt ist;
wobei der SSRI aus Fluoxetin, Paroxetin, Sertralin, Escitalopram, Citalopram oder Fluvoxamin ausgewählt ist;
wobei der 4-Hydroxyphenylpyruvat-Dioxygenase- (HPPD) Hemmer SYN-118 (Synosia Therapeutics) ist;
wobei der PDE4-Hemmer aus Ronomilast, Roflumilast, Mesembrin, Rolipram, Ibudilast, Piclamilast, Luteolin, Cilomilast, Tofimilast (9-Cyclopentyl-5,6-dihydro-7-ethyl-3-(thien-2-yl)-9H-pyrazolo(3,4-c)-1,2,4-triazolo(4,3-a)pyridin), Tipelukast, Ibudilast, Apremilast ((S)-N-[2-[1-(3-Ethoxy-4-methoxyphenyl)-2-methansulfonylethyl]-1,3-dioxo-2,3-dihydro-1H-isoindol-4-yl]acetamid, Celgene Corporation), CC-1088 (einem Thalidomid-Analogon-Hemmer von PDE4, Celgene Corporation), 8-Biarylnaphthyridinonen wie MK-0952 (Merck), DC-AT-46 (7-Benzylamino-6-chlor-2-piperazino-4-pyrrolidino-pteridin), HT-0712 ((3R,5R)-5-(3-(Cyclopentyloxy)-4-methoxyphenyl)-3-(3-methylbenzyl)piperidin-2-on, Helicon Therapeutics, Inc.), MEM-1414 (Roche), MEM-1917 (Roche), MEM-1018 (Roche), MEM-1091 (Roche) oder DG071 (decode Genetics) ausgewählt ist; und
wobei der PDE2-Hemmer BAY 60-7550 ist.

11. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit mindestens einem Antikonvulsivum verabreicht wird, das, gegebenenfalls, aus Primidon oder Zonisamid ausgewählt ist.

12. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit mindestens einem mGluR5-Antagonisten verabreicht wird, der, gegebenenfalls, AFQ056 ist.

13. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit mindestens einem L-Typ-Kalziumkanal-Blocker verabreicht wird, der, gegebenenfalls, aus Isradipin, Nimodipin oder Nilvadipin ausgewählt ist.

14. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit mindestens einem NR2B-selektiven NMDA-Antagonisten verabreicht wird, der, gegebenenfalls, aus Ro 25-6981, EVT-101 oder EVT-103 ausgewählt ist.

15. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon verabreicht wird mit (i) mindestens einem Adenosin-A2a-Rezeptor-Antagonisten, der, gegebenenfalls, aus Istradefyllin (KW-6002), SYN-115 (4-Hydroxy-N-[4-methoxy-7-(4-morpholinyl)-2-benzothiazolyl]-4-methyl-1-piperidincarboxamid) oder Preladenant (SCH420814) ausgewählt ist; oder (ii) mindestens einem Alpha-2-Adrenozeptor-Antagonisten, der, gegebenenfalls, aus Fipamezol (JP1730) oder Idazoxan ausgewählt ist.

16. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit mindestens einem 4-Hydroxyphenylpyruvat-Dioxygenase- (HPPD-) Hemmer verabreicht wird, der, gegebenenfalls, SYN-118 ist.

17. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit mindestens einem Nikotinischer-Acetylcholinrezeptor- (nAChR-) Modulator verabreicht wird, der, gegebenenfalls, aus einem selektiven oder nicht selektiven nAChR-Agonisten; einem selektiven oder nicht selektiven nAChR-Partialagonisten; einem kompetitiven nAChR-Antagonisten; einem nicht kompetitiven nAChR-Antagonisten; Nikotin (NP002); Acetylcholin; Cholin; Epibatidin; Lobelin; Vareniclin; oder Sazetidin-A ausgewählt ist.

18. Eltoprazin oder ein pharmazeutisch verträgliches Säureadditionssalz davon, zur Verwendung im Verfahren nach Anspruch 9 oder Anspruch 10, wobei das Eltoprazin oder ein pharmazeutisch verträgliches Säureadditionssalz davon mit zumindest einem Phosphodiesterase- (PDE-) Hemmer verabreicht wird, der, gegebenenfalls, ein Phosphodiesterase-2- (PDE2-) Hemmer oder ein Phosphodiesterase-4- (PDE4-) Hemmer ist.

19. Eltoprazin oder ein pharmazeutisch verträgliches Säureadditionssalz davon, zur Verwendung im Verfahren nach einem der Ansprüche 9 bis 18, wobei das Eltoprazin oder ein pharmazeutisch verträgliches Säureadditionssalz davon und der mindestens eine zusätzliche aktive Inhaltsstoff an den Patienten in einem Verabreichungsmodus verabreicht werden, der ausgewählt ist aus:
(i) Verabreichung einer Kombination von dem Eltoprazin und dem mindestens einen zusätzlichen aktiven Inhaltsstoff an den Patienten mit Bedarf daran, bevor der Patient eine Bewegungsstörung-, eine Motorikstörung-Nebenwirkung entwickelt, die mit der Verabreichung von Levodopa oder einem Dopamin-verwandten Wirkstoff an einen menschlichen adulten Patienten, der Morbus Parkinson hat, assoziiert ist; oder
(ii) Verabreichung einer Kombination von dem Eltoprazin und dem mindestens einen zusätzlichen aktiven Inhaltsstoff an den Patienten mit Bedarf daran, nachdem der Patient eine Bewegungsstörung-, eine Motorikstörung-Nebenwirkung entwickelt hat, die mit der Verabreichung von Levodopa oder einem Dopamin-verwandten Wirkstoff an einen menschlichen adulten Patienten, der Morbus Parkinson hat, assoziiert ist.

20. Eltoprazin oder ein pharmazeutisch verträgliches Säureadditionssalz davon, zur Verwendung im Verfahren nach einem der Ansprüche 9 bis 19, wobei das Eltoprazin an den Patienten in einem Dosierungsregime verabreicht wird, das ausgewählt ist aus:
(i) Verabreichung des Eltoprazins an den Patienten, bevor das mindestens eine zusätzliche aktive Mittel verabreicht wird;
(ii) Verabreichung des Eltoprazins an den Patienten, nachdem das mindestens eine zusätzliche aktive Mittel verabreicht ist;
(iii) Verabreichung des Eltoprazins an den Patienten gleichzeitig mit der Verabreichung des mindestens einen zusätzlichen aktiven Mittels.

21. Eltoprazin oder ein pharmazeutisch verträgliches Säureadditionssalz davon nach Anspruch 1, in Kombination mit tiefer Hirnstimulation und/oder transkranieller Magnetstimulation.

22. Eltoprazin oder ein pharmazeutisch verträgliches Säureadditionssalz davon nach Anspruch 21, wobei die Reihenfolge der Behandlung ein Verabreichungsregime umfasst, das ausgewählt ist aus:
(i) die tiefe Hirnstimulation und/oder transkranielle Magnetstimulation geht der Behandlung des Patienten mit Levodopa oder einem Dopamin-verwandten Wirkstoff voraus;
(ii) die tiefe Hirnstimulation und/oder transkranielle Magnetstimulation folgt der Behandlung des Patienten mit Levodopa oder einem Dopamin-verwandten Wirkstoff;
(iii) die tiefe Hirnstimulation und/oder transkranielle Magnetstimulation geht der Behandlung des Patienten mit Eltoprazin oder einem pharmazeutisch verträglichen Säureadditionssalz davon voraus; oder
(iv) die tiefe Hirnstimulation und/oder transkranielle Magnetstimulation folgt der Behandlung des Patienten mit Eltoprazin oder einem pharmazeutisch verträglichen Säureadditionssalz davon.

23. Eltoprazin oder ein pharmazeutisch verträgliches Säureadditionssalz davon nach einem der Ansprüche 1 bis 22, wobei der Dopamin-verwandte Wirkstoff ausgewählt ist aus einem Dopamin-Rezeptor-Agonisten, der, gegebenenfalls, Bromocriptin, Pergolid, Cabergolin, Apomorphin oder Lisurid ist; oder einem nicht ergolinen Dopamin-Agonisten, der, gegebenenfalls, Ropinirol oder Pramipexol ist.

24. Eltoprazin oder ein pharmazeutisch verträgliches Säureadditionssalz davon, zur Verwendung in einem Verfahren nach einem der Ansprüche 1 bis 8, wobei das Verfahren Verabreichen, an den Patienten, einer Dosis Levodopa, einer Dosis Carbidopa und einer Dosis Eltoprazin oder eines pharmazeutisch verträglichen Säureadditionssalzes davon umfasst.

25. Eltoprazin oder ein pharmazeutisch verträgliches Säureadditionssalz davon, zur Verwendung in einem Verfahren zur Verhütung, Linderung und/oder Behandlung einer Motorikstörung-Nebenwirkung, die mit der Verabreichung von Levodopa oder einem Dopamin-verwandten Wirkstoff an einen menschlichen adulten Patienten, der Morbus Parkinson hat, assoziiert ist, nach einem der Ansprüche 1 bis 8, wobei das Verfahren Verabreichen, an den Patienten, einer Dosis Levodopa, einer Dosis Carbidopa, einer Dosis Entacapon und einer Dosis Eltoprazin oder eines pharmazeutisch verträglichen Säureadditionssalzes davon umfasst.

26. Eltoprazin oder ein pharmazeutisch verträgliches Säureadditionssalz davon in Kombination mit Levodopa oder einem Dopamin-verwandten Wirkstoff, zur Verwendung in einem Verfahren zur Verhütung, Linderung und/oder Behandlung eines menschlichen adulten Patienten, der Morbus Parkinson hat; wobei das Verfahren Verabreichen, an den menschlichen adulten Patienten mit Morbus Parkinson, mit Behandlungsbedarf, von Levodopa oder einem Dopamin-verwandten Wirkstoff und Eltoprazin oder einem pharmazeutisch verträglichen Säureadditionssalz davon in einem Dosisbereich von 5 mg/Tag bis 7,5 mg/Tag umfasst.

27. Eltoprazin oder ein pharmazeutisch verträgliches Säureadditionssalz davon nach einem der Ansprüche 1 bis 26, wobei das Eltoprazin in einer Dosis von 5 mg/Tag verabreicht wird.

28. Eltoprazin oder ein pharmazeutisch verträgliches Säureadditionssalz davon nach einem der Ansprüche 1 bis 26, wobei das Eltoprazin in einer Dosis von 7 mg/Tag verabreicht wird.

29. Eltoprazin oder ein pharmazeutisch verträgliches Säureadditionssalz davon nach einem der Ansprüche 1 bis 26, wobei das Eltoprazin in einer Dosis von 7,5 mg/Tag verabreicht wird.

## Revendications

1. Eltoprazine ou un sel d'addition acide pharmaceutiquement acceptable de celle-ci pour une utilisation dans une méthode de prévention, atténuation et/ou traitement d'un effet secondaire de trouble du mouvement ou de trouble moteur associé à l'administration de lévodopa ou d'un médicament apparenté à la dopamine à un patient adulte humain souffrant de la maladie de Parkinson; la méthode comprenant l'administration audit patient adulte humain souffrant de la maladie de Parkinson ayant besoin d'un traitement, d'eltoprazine ou d'un sel d'addition acide pharmaceutiquement acceptable de celle-ci à une gamme de dose de 5 mg/jour à 7,5 mg/jour.

2. Eltoprazine ou un sel d'addition acide pharmaceutiquement acceptable de celle-ci pour une utilisation dans la méthode selon la revendication 1, où ledit effet secondaire de trouble du mouvement ou de trouble moteur est une dyskinésie, une hyperkinésie ou une dyskinésie induite par la lévodopa (LID).

3. Eltoprazine ou un sel d'addition acide pharmaceutiquement acceptable de celle-ci pour une utilisation dans une méthode de prévention, atténuation et/ou traitement d'une dyskinésie induite par la lévodopa (LID), la méthode comprenant l'administration à un patient adulte humain souffrant de la maladie de Parkinson ayant besoin d'un traitement, d'eltoprazine ou d'un sel d'addition acide pharmaceutiquement acceptable de celle-ci à une gamme de dose de 5 mg/jour à 7,5 mg/jour.

4. Eltoprazine ou un sel d'addition acide pharmaceutiquement acceptable de celle-ci, pour une utilisation dans une méthode selon l'une quelconque des revendications 1 à 3, la méthode comprenant l'administration audit patient d'une dose de lévodopa et d'une dose d'eltoprazine ou d'un sel d'addition acide pharmaceutiquement acceptable de celle-ci, où ladite eltoprazine est administrée à une gamme de dose de 5 mg/jour à 7,5 mg/jour.

5. Eltoprazine ou un sel d'addition acide pharmaceutiquement acceptable de celle-ci pour une utilisation dans la méthode selon la revendication 4, où ladite eltoprazine et ladite lévodopa sont simultanément actives chez ledit humain.

6. Eltoprazine ou un sel d'addition acide pharmaceutiquement acceptable de celle-ci pour une utilisation dans la méthode selon la revendication 4 ou la revendication 5, où ladite eltoprazine est administrée à l'humain avant que ledit humain développe une dyskinésie associée à l'administration de ladite lévodopa.

7. Eltoprazine ou un sel d'addition acide pharmaceutiquement acceptable de celle-ci pour une utilisation dans la méthode selon la revendication 4 ou la revendication 5, où ladite eltoprazine est administrée audit humain après que ledit humain développe une dyskinésie associée à l'administration de ladite lévodopa.

8. Eltoprazine ou un sel d'addition acide pharmaceutiquement acceptable de celle-ci pour une utilisation dans la méthode selon l'une quelconque des revendications 4 à 7, où ladite eltoprazine est administrée à l'humain avant l'administration de ladite lévodopa.

9. Eltoprazine ou un sel d'addition acide pharmaceutiquement acceptable de celle-ci pour une utilisation dans la méthode selon l'une quelconque des revendications 1 à 8, où ladite eltoprazine est administrée en combinaison avec au moins un ingrédient actif supplémentaire, où le au moins un ingrédient actif supplémentaire est choisi dans le groupe constitué de:
un inhibiteur du complexe (mTOR) 1 (mTORC1);
un antagoniste de 5-HTla/2a/alpha-2;
un agoniste inverse de 5-HT2a;
un antagoniste de récepteur A2a d'adénosine;
un antagoniste de récepteur adrénergique alpha-1 ;
un antagoniste de récepteur adrénergique alpha-2;
un antagoniste d'AMPA;
un antagoniste d'AMPA/kaïnate;
un anticonvulsivant;
un antagoniste bêta-2;
un agoniste de CB;
un antagoniste de D2/5-HT2A+;
un stabilisant dopaminergique;
un inhibiteur de libération de glutamate/amplificateur de la fonction dopaminergique;
une combinaison d'un inhibiteur de MAO-B et d'un inhibiteur d'acétylcholinestérase;
un inhibiteur d'acétylcholinestérase;
un inhibiteur de cholinestérase qui inhibe à la fois une butyrylcholinestérase et une acétylcholine stérase ;
un antagoniste de H2;
un inhibiteur d'hydroxylase kynurénique;
un antagoniste de Ca2+ de type L ou un bloqueur des canaux calciques de type L;
un antagoniste de mGluR5;
un inhibiteur de réabsorption de monoamine;
un agoniste de récepteur opioïde mu;
un modulateur de récepteur d'acétylcholine nicotinique (nAChR);
un inhibiteur d'oxyde nitrique synthase;
un composé nootrope;
un antagoniste de NMDA NR1A/2B;
un antagoniste de NMDA sélectif NR2B;
un acide gras oméga-3;
un antagoniste des opiacés;
un anticholinergique;
un SSRI (inhibiteur de réabsorption de sérotonine sélectif);
un inhibiteur de 4-hydroxyphénylpyruvate dioxygénase (HPPD);
un inhibiteur de phosphodiestérase (PDE) tel qu'un inhibiteur de phosphodiestérase 4 (PDE4) ou un inhibiteur de phosphodiestérase 2 (PDE2); et
la carbidopa.

10. Eltoprazine ou un sel d'addition acide pharmaceutiquement acceptable de celle-ci pour une utilisation dans la méthode selon la revendication 9,
où l'inhibiteur de complexe (mTOR) 1 (mTORC1) est la rapamycine;
où l'antagoniste de 5-HT1a/2a/alpha-2 est la mirtazapine;
où l'agoniste inverse de 5-HT2a est choisi parmi ACP103 (pimavansérine), nélotansérine, éplivansérine ou AC-90179 [chlorhydrate de 2-(4-méthoxyphényl)-N-(4-méthyl-benzyl)-N-(1-méthyl-pipéridin-4-yl)-acétamide]);
où l'antagoniste de récepteur A2a d'adénosine est choisi parmi istradéfylline (KW-6002), SYN-115 (4-hydroxy-N-[4-méthoxy-7-(4-morpholinyl)-2-benzothiazolyl]-4-méthyl-1-pipéridinecarboxamide, (Synosia Therapeutics), préladenant (SCH420814), ZM-241,385 (4-(2-[7-amino-2-(2-furyl)[1,2,4]-triazolo[2,3-a][1,3,5]triazin-5-ylamino]éthyl)phénol), VER-7835 (2-amino-6-(furan-2-yl)-N-(thiophén-2-ylméthyl)-9H-purine-9-carboxamide), MSX-3 (3-(3-hydroxypropyl)-7- méthyl-8-(m-méthoxystyryl)-1-propargylxanthine) ou ST-1535 (2-butyl-9-méthyl-8-(2H-1,2,3-triazol-2-yl)-9H-purin-6-ylamine));
où l'antagoniste de récepteur adrénergique alpha-1 est choisi parmi HEAT (2-[[bêta-(-4-hydroxyphényl)éthyl]aminométhyl]-1-tétralone), alfuzosine, doxazosine, moxisylyte, prazosine ou trimazosine;
où l'antagoniste de récepteur adrénergique alpha-2 est choisi parmi idazoxan, fipamézole (JP1730), atipamézole, miansérine, phentolamine ou tolazoline;
où l'antagoniste d'AMPA est E2007 (pérampanel);
où l'antagoniste d'AMPA/kaïnate est LY300164 [7-acétyl-5-(4-aminophényl)-8,9-dihydro-8-méthyl-7H-1,3-dioxolo(4,5H)-2,3-benzodiazépine];
où l'anticonvulsivant est choisi parmi primidone, zonisamide, lévétiracétam, mesuximide, acétate d'eslicarbazépine ou prégabaline;
où l'antagoniste bêta-2 est choisi parmi propranolol, alprénolol, cartéolol, nadolol, sotalol ou timolol;
où l'agoniste de CB est choisi parmi nabilone ou WIN55,212-2 (mésylate de (R)-(+)-[2,3-dihydro-5-méthyl-3-(4-morpholinylméthyl)pyrrolo[1,2,3-de]-1,4-benzoxazin-6-yl] -1 -naphtalénylméthanone) ;
où l'antagoniste de D2/5-HT2A+ est clozapine ou quétiapine;
où le stabilisant dopaminergique est choisi parmi ACR325, aripiprazole ou pridopidine;
où l'inhibiteur de libération de glutamate/amplificateur de la fonction dopaminergique est le safinamide;
où la combinaison de l'inhibiteur de MAO-B et de l'inhibiteur d'acétylcholinestérase est choisie parmi rasagiline, sélégiline, safinamide ou EVT-302 (Evotec AG) en combinaison avec ladostigil (TV-3326);
où l'inhibiteur d'acétylcholinestérase est le donépézil;
où l'inhibiteur de cholinestérase qui inhibe à la fois la butyrylcholinestérase et l'acétylcholinestérase est la rivastigmine;
où l'antagoniste de H2 est choisi parmi famotidine, cimétidine, burimamide, ranitidine ou métiamide;
où l'inhibiteur d'hydroxylase kynurénique est choisi parmi Ro 61-8048 (3,4-diméthoxy-N-[4-(3-nitrophényl)-2-thiazolyl]benzènesulfonamide ou FCE 28833 [acide (R,S)-2-amino-4-oxo-4-(3',4'-dichlorophényl)butanoïque];
où l'antagoniste de Ca2+ de type L ou le bloqueur des canaux calciques de type L est choisi parmi isradipine, amlodipine, azelnidipine, cilnidipine, lacidipine, nimodipine, nilvadipine ou topiramate;
où l'antagoniste de mGluR5 est AFQ056 (6-méthyl-2-(phényléthynyl)pyridine);
où l'inhibiteur de réabsorption de monoamine est choisi parmi NS2330 (tesofensine), bicifadine ou DOV-102,677 [(5R)-5-(3,4-dichlorophényl)bicyclo[3.1.0]hexane] ;
où le modulateur de récepteur d'acétylcholine nicotinique (nAChR) est choisi parmi un agoniste de nAChR sélectif ou non sélectif; un agoniste partiel de nAChR sélectif ou non sélectif; un antagoniste de nAChR compétitif; un antagoniste de nAChR non compétitif; nicotine (NP002); acétylcholine; choline; épibatidine; lobéline; varénicline; sazétidine-A; DMXB-A (GTS-21, 3-2,4 diméthoxybenzylidène); ou SIB-1508Y (3-éthynyl-5-[(2S)-1-méthyl-2-pyrrolidinyl]pyridine);
où l'antagoniste nicotinique est choisi parmi chlorisondamine, d-tubocurarine, mécamylamine ou bupropion;
où l'inhibiteur d'oxyde nitrique synthase est choisi parmi 7-nitroindazole ou NG-nitro-L-arginine;
où le composé nootrope est le lévitircétam;
où l'antagoniste de NMDA NR1A/2B est Cl-1041 (5-[2-[4-(4-fluorobenzyl)pipéridin-1-yl]éthylsulfinyl]-2,3-dihydro-1H-benzimidazol-2-one));
où l'antagoniste de NMDA sélectif NR2B est choisi parmi MK0657, CP-101,606 (traxoprodil), éliprodil, Ro 25-6981 ((±)-(R*,S*)-α-(4-hydroxyphényl)-β-méthyl-4-(phénylméthyl)-1-pipéridine propanol), EVT-101 (Evotec AG) ou EVT-103 (Evotec)
où l'acide gras oméga-3 est l'acide docosahexaénoïque);
où l'antagoniste des opiacés est la naltrexone;
où l'anticholinergique est choisi parmi amantadine, bromure d'ipratropium, bromure d'oxytropium ou tiotropium;
où le SSRI est choisi parmi fluoxétine, paroxétine, sertraline, escitalopram, citalopram ou fluvoxamine;
où l'inhibiteur de 4-hydroxyphénylpyruvate dioxygénase (HPPD) est SYN-118, (Synosia Therapeutics);
où l'inhibiteur de PDE4 est choisi parmi ronomilast, roflumilast, mésembrine, rolipram, ibudilast, piclamilast, lutéoline, cilomilast, tofimilast (9-cyclopentyl-5,6-dihydro-7-éthyl-3-(thién-2-yl)-9H-pyrazolo(3,4-c)-1,2,4-triazolo(4,3-a)pyridine), tipélukast, ibudilast, aprémilast ((S)-N-[2-[1-(3-éthoxy-4-méthoxyphényl)-2-méthanesulfonyléthyl]-1,3-dioxo-2,3-dihydro-1H-isoindol-4-yl]acétamide, Celgene Corporation), CC-1088 (un inhibiteur de PDE4 analogue de thalidomide, Celgene Corporation), 8-biarylnaphtyridinones telles que MK-0952 (Merck), DC-AT-46 (7-benzylamino-6-chloro-2-pipérazino-4-pyrrolidino-ptéridine), HT-0712 ((3R,5R)-5-(3-(cyclopentyloxy)-4-méthoxyphényl)-3-(3-méthylbenzyl)pipéridin-2-one, Helicon Therapeutics, Inc.), MEM-1414 (Roche), MEM-1917 (Roche), MEM-1018 (Roche), MEM-1091 (Roche) ou DG071 (decode Genetics); et
où l'inhibiteur de PDE2 est BAY 60-7550.

11. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un anticonvulsivant, qui est, optionnellement, choisi parmi primidone ou zonisamide.

12. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un antagoniste de mGluR5, qui est, optionnellement, AFQ056.

13. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un bloqueur des canaux calciques de type L, qui est, optionnellement, choisi parmi isradipine, nimodipine ou nilvadipine.

14. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un antagoniste de NMDA sélectif NR2B, qui est, optionnellement, choisi parmi Ro 25-6981, EVT-101 ou EVT-103.

15. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec (i) au moins un antagoniste de récepteur A2a d'adénosine, qui est, optionnellement, choisi parmi istradéfylline (KW-6002), SYN-115 (4-hydroxy-N-[4-méthoxy-7-(4-morpholinyl)-2-benzothiazolyl]-4-méthyl-1-pipéridinecarboxamide ou préladenant (SCH420814); ou (ii) au moins un antagoniste de récepteur adrénergique alpha-2, qui est, optionnellement, choisi parmi fipamézole (JP1730) ou idazoxan.

16. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un inhibiteur de 4-hydroxyphénylpyruvate dioxygénase (HPPD), qui est, optionnellement, SYN-118.

17. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un modulateur de récepteur d'acétylcholine nicotinique (nAChR), qui est, optionnellement, choisi parmi un agoniste de nAChR sélectif ou non sélectif; un agoniste partiel de nAChR sélectif ou non sélectif; un antagoniste de nAChR compétitif; un antagoniste de nAChR non compétitif; nicotine (NP002); acétylcholine; choline; épibatidine; lobéline; varénicline; ou sazétidine-A.

18. Eltoprazine ou un sel d'addition acide pharmaceutiquement acceptable de celle-ci pour une utilisation dans la méthode selon la revendication 9 ou la revendication 10, où ladite eltoprazine ou un sel d'addition acide pharmaceutiquement acceptable de celle-ci est administré avec au moins un inhibiteur de phosphodiestérase (PDE), qui est, optionnellement, un inhibiteur de phosphodiestérase 2 (PDE2) ou un inhibiteur de phosphodiestérase 4 (PDE4).

19. Eltoprazine ou un sel d'addition acide pharmaceutiquement acceptable de celle-ci pour une utilisation dans la méthode selon l'une quelconque des revendications 9 à 18, où ladite eltoprazine ou un sel d'addition acide pharmaceutiquement acceptable de celle-ci et ledit au moins un ingrédient actif supplémentaire sont administrés audit patient dans un mode d'administration choisi parmi:
(i) administration d'une combinaison de ladite eltoprazine et dudit au moins un ingrédient actif supplémentaire audit patient ayant besoin de celle-ci avant que ledit patient développe un effet secondaire de trouble du mouvement, de trouble moteur associé à l'administration de lévodopa ou d'un médicament apparenté à la dopamine à un patient adulte humain souffrant de la maladie de Parkinson; ou
(ii) administration d'une combinaison de ladite eltoprazine et dudit au moins un ingrédient actif supplémentaire audit patient ayant besoin de celle-ci après que ledit patient développe un effet secondaire de trouble du mouvement, de trouble moteur associé à l'administration de lévodopa ou d'un médicament apparenté à la dopamine à un patient adulte humain souffrant de la maladie de Parkinson.

20. Eltoprazine ou un sel d'addition acide pharmaceutiquement acceptable de celle-ci pour une utilisation dans la méthode selon l'une quelconque des revendications 9 à 19, où ladite eltoprazine est administrée au patient dans un schéma d'administration choisi parmi:
(i) administration de ladite eltoprazine au patient avant l'administration dudit au moins un agent actif supplémentaire;
(ii) administration de ladite eltoprazine au patient après l'administration dudit au moins un agent actif supplémentaire;
(iii) administration de ladite eltoprazine au patient simultanément à l'administration dudit au moins un agent actif supplémentaire.

21. Eltoprazine ou un sel d'addition acide pharmaceutiquement acceptable de celle-ci selon la revendication 1 en combinaison avec une stimulation cérébrale profonde et/ou une stimulation magnétique transcrânienne.

22. Eltoprazine ou un sel d'addition acide pharmaceutiquement acceptable de celle-ci selon la revendication 21, où l'ordre dudit traitement comprend un schéma d'administration choisi parmi:
(i) ladite stimulation cérébrale profonde et/ou ladite stimulation magnétique transcrânienne précèdent le traitement du patient avec la lévodopa ou un médicament apparenté à la dopamine;
(ii) ladite stimulation cérébrale profonde et/ou ladite stimulation magnétique transcrânienne suivent le traitement du patient avec la lévodopa ou un médicament apparenté à la dopamine;
(iii) ladite stimulation cérébrale profonde et/ou ladite stimulation magnétique transcrânienne précèdent ledit traitement du patient avec l'eltoprazine ou un sel d'addition acide pharmaceutiquement acceptable de celle-ci; ou
(iv) ladite stimulation cérébrale profonde et/ou ladite stimulation magnétique transcrânienne suivent ledit traitement du patient avec l'eltoprazine ou un sel d'addition acide pharmaceutiquement acceptable de celle-ci.

23. Eltoprazine ou un sel d'addition acide pharmaceutiquement acceptable de celle-ci selon l'une quelconque des revendications 1 à 22, où ledit médicament apparenté à la dopamine est choisi parmi un agoniste de récepteur de dopamine, qui est, optionnellement, bromocriptine, pergolide, cabergoline, apomorphine ou lisuride; ou un agoniste de dopamine non ergoline, qui est, optionnellement, ropinirole ou pramipexole.

24. Eltoprazine ou un sel d'addition acide pharmaceutiquement acceptable de celle-ci, pour une utilisation dans une méthode selon l'une quelconque des revendications 1 à 8, la méthode comprenant l'administration audit patient d'une dose de lévodopa, d'une dose de carbidopa et d'une dose d'eltoprazine ou d'un sel d'addition acide pharmaceutiquement acceptable de celle-ci.

25. Eltoprazine ou un sel d'addition acide pharmaceutiquement acceptable de celle-ci, pour une utilisation dans une méthode de prévention, atténuation et/ou traitement d'un effet secondaire de trouble moteur associé à l'administration de lévodopa ou d'un médicament apparenté à la dopamine à un patient adulte humain souffrant de la maladie de Parkinson, selon l'une quelconque des revendications 1 à 8, la méthode comprenant l'administration audit patient d'une dose of lévodopa, d'une dose de carbidopa, d'une dose d'entacapone et d'une dose d'eltoprazine ou d'un sel d'addition acide pharmaceutiquement acceptable de celle-ci.

26. Eltoprazine ou un sel d'addition acide pharmaceutiquement acceptable de celle-ci en combinaison avec la lévodopa ou un médicament apparenté à la dopamine, pour une utilisation dans une méthode de prévention, atténuation et/ou traitement d'un patient adulte humain souffrant de la maladie de Parkinson; la méthode comprenant l'administration audit patient adulte humain souffrant de la maladie de Parkinson ayant besoin d'un traitement, de lévodopa ou d'un médicament apparenté à la dopamine et d'eltoprazine ou d'un sel d'addition acide pharmaceutiquement acceptable de celle-ci à une gamme de dose de 5 mg/jour à 7,5 mg/jour.

27. Eltoprazine ou un sel d'addition acide pharmaceutiquement acceptable de celle-ci selon l'une quelconque des revendications 1 à 26, où ladite eltoprazine est administrée à une dose de 5 mg/jour.

28. Eltoprazine ou un sel d'addition acide pharmaceutiquement acceptable de celle-ci selon l'une quelconque des revendications 1 à 26, où ladite eltoprazine est administrée à une dose de 7 mg/jour.

29. Eltoprazine ou un sel d'addition acide pharmaceutiquement acceptable de celle-ci selon l'une quelconque des revendications 1 à 26, où ladite eltoprazine est administrée à une dose de 7,5 mg/jour.
